# EUROPEAN PATENT APPLICATION

(11) **EP 4 371 989 A1**
(43) Date of publication of application: **22.05.2024**
(21) Application number: 22840996.7
(22) Date of filing: 22.04.2022
(51) Int. Cl.: C07D 495/04, H10K 99/00, H10K 50/00, H10K 85/00

(54) **ORGANIC COMPOUND CONTAINING HETEROCYCLIC RING, AND MIXTURE AND ORGANIC ELECTRONIC DEVICE**

(30) Priority: 12.07.2021 CN 202110784797
(71) Applicant: Guangzhou Chinaray Optoelectronic Materials Ltd., Guangzhou, Guangdong 510663 (CN)
(72) Inventor: HE, Ruifeng, Guangzhou, Guangdong 510663 (CN); WU, Canjie, Guangzhou, Guangdong 510663 (CN); WU, Xuexiong, Guangzhou, Guangdong 510663 (CN); SONG, Jingyao, Guangzhou, Guangdong 510663 (CN)
(74) Representative: Nederlandsch Octrooibureau
(86) International application number: PCT/CN2022/088453
(87) International publication number: WO 2023/284352

(57) **Abstract**

The present application discloses heterocyclic-containing organic compounds, mixtures and organic electronic devices. The heterocyclic group-containing organic compounds in the present application have good performance of transferring and adjusting charge balance, and can be used as host materials in an organic electronic device, thereby improving the luminous efficiency and the lifetime of the device.

## Description

### TECHNICAL FIELD

The present application relates to the technical field of organic photovoltaic materials, and more particularly, to heterocycle-containing organic compounds, mixtures, and organic electronic devices.

### BACKGROUND

Organic photovoltaic materials have diversity in synthesis, have relatively low manufacturing cost, and excellent optical and electrical properties. Organic light-emitting Diodes (OLED) have broad development potential due to wide viewing angle, fast response time, low working voltage and thin panel thickness, and are used in the preparation of optoelectronic devices, such as flat panel displays and illumination.

The organic electroluminescent phenomenon refers to a phenomenon of converting electrical energy to photonic energy with organic substances. An organic electroluminescent element utilizing the organic electroluminescent phenomenon usually has a structure including an anode, a cathode, and an organic functional layer therebetween. In order to improve the efficiency and lifetime of the organic electroluminescent element, the organic functional layer has a multi-layer structure, and each layer includes different organic substances. For example, each layer can be a hole injection layer, a hole transport layer, a light-emitting auxiliary layer, a light-emitting layer, an electron transport layer, an electron injection layer, etc. When a voltage is applied between two electrodes of such an organic electroluminescent element, holes are injected into the organic functional layer from the anode, electrons are injected into the organic functional layer from the cathode; and an exciton is formed when an injected hole and an injected electron recombine in the emission layer. The exciton emits light when it transitions back to the ground state. The organic electroluminescent element has characteristics of self-emission, high luminance, high efficiency, low driving voltage, wide viewing angle, and high contrast, etc.

Currently, materials of the light-emitting layer usually adopt a form of doping a host material with a guest material, and holes and electrons are transported through the combination of host materials and guest materials, thereby improving the lifetime and efficiency of the devices. As a result, the host materials play an important role in organic electroluminescent devices. Currently, although a large number of host materials have been developed, there are still some problems in the corresponding devices, such as unbalanced carrier transport and insufficient device lifetime. How to design new host materials with better performance to improve the efficiency and lifetime of the device has been a problem urgently to be solved by a person skilled in the art.

### TECHNICAL PROBLEMS

The efficiency and lifetime of devices in related arts need to be further improved.

### TECHNICAL SOLUTIONS

The present application provides heterocycle-containing organic compounds, mixtures, and organic electronic devices.

Technical solutions of the present application are as follows:

A heterocyclic group-containing organic compound, wherein the heterocyclic group-containing organic compound has a structure represented by formula (1) or (2): 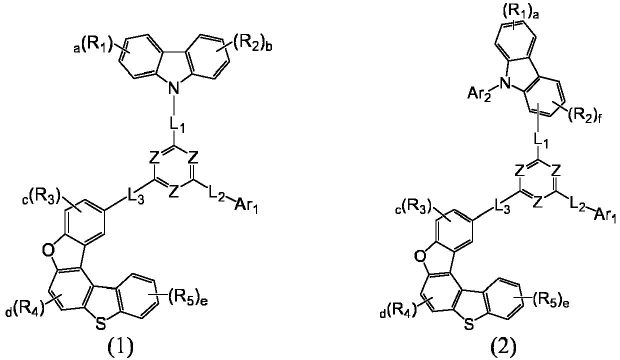 wherein:
Z is independently selected from CR₆ or an N atom, wherein at least one Z is an N atom;
R₁, R₂ or R₆ are each independently selected from -H, -D, or a linear alkyl group having 1 to 20 carbon atoms, a linear alkoxy group having 1 to 20 carbon atoms, or a linear thioalkoxy group having 1 to 20 carbon atoms, or a branched alkyl group having 3 to 20 carbon atoms, or a cyclic alkyl group having 3 to 20 carbon atoms, or a branched alkoxy group having 3 to 20 carbon atoms, or a cyclic alkoxy group having 3 to 20 carbon atoms, or a branched thioalkoxy group having 3 to 20 carbon atoms, or a cyclic thioalkoxy group having 3 to 20 carbon atoms, or a silyl group, or a ketone group having 1 to 20 carbon atoms, or an alkoxycarbonyl group having 2 to 20 carbon atoms, or an aryloxycarbonyl group having 7 to 20 carbon atoms, a cyano group, a carbamoyl group, a haloformyl group, a formyl group, an isocyano group, an isocyanate group, a thiocyanate group or an isothiocyanate group, a hydroxyl group, a nitro group, -CF₃, -Cl, -Br, -F, -I, or substituted or unsubstituted aromatic group having 6 to 60 ring atoms, or a substituted or unsubstituted heteroaromatic groups having 5 to 60 ring atoms, or a substituted or unsubstituted aryloxy group or heteroaryloxy group having 5 to 60 ring atoms, or a combinations thereof;
R₃, R₄ or R₅ are each independently selected from -H, -D, or a linear alkyl group having 1 to 20 carbon atoms, or a branched or cyclic alkyl group having 3 to 20 carbon atoms, or a phenyl group;
An is selected from a substituted or unsubstituted aromatic group having 6 to 30 ring atoms, or a substituted or unsubstituted heteroaromatic group having 5 to 30 ring atoms;
Ar₂ is selected from -H, -D, or a linear alkyl group having 1 to 20 carbon atoms, or a branched or cyclic alkyl group having 3 to 20 carbon atoms, or a substituted or unsubstituted aromatic group having 6 to 30 ring atoms, or a substituted or unsubstituted heteroaromatic group having 5 to 30 ring atoms;
L₁, L₂ and L₃ are each independently selected from a single bond, or a substituted or unsubstituted aromatic group having 6 to 30 ring atoms, or a substituted or unsubstituted heteroaromatic group having 5 to 30 ring atoms; and
a, b and e are each independently selected from 0, 1, 2, 3, or 4; d is selected from 0, 1 or 2; and c and f are each independently selected from 0, 1, 2 or 3.

The present application further provides a mixture including at least one heterocyclic group-containing organic compound and at least one organic functional material. The organic functional material is selected from a hole injection material, a hole transport material, an electron transport material, an electron injection material, an electron blocking material, a hole blocking material, a light-emitting material, a host material, or an organic dye.

The present application further provides an organic electronic device, which includes a first electrode, a second electrode and at least one organic functional layer disposed between the first electrode and the second electrode. The organic functional layer includes at least one of the above-mentioned heterocyclic group-containing organic compounds.

### BENEFICIAL EFFECTS

In the present application, the heterocyclic group-containing organic compounds represented by formula (1) or (2) have good performance of transferring and adjusting charge balance, and can be used as host materials in an organic electronic device, thereby improving the luminous efficiency and the lifetime of the device.

### EMBODIMENTS OF THE PRESENT APPLICATION

It can be understood that H in the structural formula of the above-mentioned aromatic amine organic compounds may be further substituted.

Hereinafter, technical solutions in embodiments of the present application will be clearly and completely described with reference to the embodiments of the present application. Obviously, the described embodiments are part of, but not all of, the embodiments of the present application. All the other embodiments, obtained by a person with ordinary skill in the art on the basis of the embodiments in the present application without expenditure of creative labor, belong to the protection scope of the present application.

The present application provides heterocycle-containing organic compounds, mixtures, compositions, and organic electronic devices. Detailed descriptions are given below. It should be noted that the order in which the following embodiments are described is not intended to limit the preferred order of the embodiments.

In the present application, "substituted" means that the hydrogen atom/hydrogen atoms in the substituent is/are substituted by one or more substituents.

In the present application, when the same substituent occurs multiple times, it may be independently selected from different groups, for example, when the formula contains a plurality of R1, then R1 may be independently selected from different groups.

In the present application, "substituted or unsubstituted" means that the defined group may be substituted or may not be substituted. When a defined group is substituted, it is understood that it is optionally substituted by groups acceptable in the art, including, but not limited to, D (deuterium), C₁ to C₃₀ alkyl group, heterocyclyl group having 3 to 20 ring atoms, aryl group having 6 to 20 ring atoms, heteroaryl group having 5 to 20 ring atoms, silyl group, carbonyl group, alkoxycarbonyl group, aryloxycarbonyl group, carbamoyl group, haloformyl group, formyl group, -NRR', cyano group, isocyano group, isocyanate group, thiocyanate group, isothiocyanate group, hydroxyl group, trifluoromethyl group, nitro group, or halogen, and the above groups may be further substituted by substituents acceptable in the art. It can be understood that R and R' in -NRR' are each independently substituted by groups acceptable in the art, including, but not limited to, H, D, C₁ to C₆ alkyl group, cycloalkyl group having 3 to 8 ring atoms, heterocyclyl group having 3 to 8 ring atoms, aryl group having 6 to 20 ring atoms, or heteroaryl group having 5 to 10 ring atoms. Said C₁ to C₆ alkyl group, cycloalkyl group having 3 to 8 ring atoms, heterocyclyl group having 3 to 8 ring atoms, aryl group having 6 to 20 ring atoms, or heteroaryl group having 5 to 10 ring atoms are optionally further substituted by one or more of the following groups: C₁ to C₆ alkyl group, cycloalkyl group having 3 to 8 ring atoms, heterocyclyl group having 3 to 8 ring atoms, halogen, hydroxy, nitro or amino group.

In the present application, "the number of ring atoms" means the number of atoms constituting the ring itself of a structural compound (e.g., a monocyclic compound, a fused ring compound, a crosslinked compound, a carbocyclic compound, a heterocyclic compound) obtained by synthesizing the ring by atomic bonds. In a case that the ring is replaced by a substituent, the atoms contained in the substituent are not included in the ring-forming atoms. The "number of ring atoms" mentioned below has the same meaning unless otherwise specified, for example, the number of ring atoms of benzene ring is 6, the number of ring atoms of naphthalene ring is 10, and the number of ring atoms of thienyl group is 5.

In the present application, "alkyl group" may represent a linear, branched and/or cyclic alkyl group. The number of carbons in an alkyl group can be 1 to 50, 1 to 30, 1 to 20, 1 to 10, or 1 to 6. The phrase containing the term, for example, "C₁₋₉ alkyl group" refers to an alkyl group containing 1 to 9 carbon atoms, which may independently be a C₁ alkyl group, a C₂ alkyl group, a C₃ alkyl group, a C₄ alkyl group, a C₅ alkyl group, a C₆ alkyl group, a C₇ alkyl group, a C₈ alkyl group, or a C₉ alkyl group. Non-limiting examples of alkyl groups include methyl, ethyl, n-propyl, isopropyl, n-butyl, sec-butyl, tert-butyl, isobutyl, 2-ethylbutyl, 3,3-dimethylbutyl, n-pentyl, isopentyl, neopentyl, tert-pentyl, cyclopentyl, 1-methylpentyl, 3-methylpentyl, 2-ethylpentyl, 4-methyl-2-pentyl, n-hexyl, 1-methylhexyl, 2-ethylhexyl, 2-butylhexyl, cyclohexyl, 4-methylcyclohexyl, 4-tert-butylcyclohexyl, n-heptyl, 1-methylheptyl, 2,2-dimethylheptyl, 2-ethylheptyl, 2-butylheptyl, n-octyl, tert-octyl, 2-ethyloctyl, 2-butyloctyl, 2-hexyloctyl, 3,7-dimethyloctyl, cyclooctyl, n-nonyl, n-decyl, adamantyl, 2-ethyldecyl, 2-butyldecyl, 2-hexyldecyl, 2-octyldecyl, n-undecyl, n-dodecyl, 2-ethyldodecyl, 2-butyldodecyl, 2-hexyldodecyl, 2-octyldodecyl, n-tridecyl, n-tetradecyl, n-pentadecyl, n-hexadecyl, 2-ethylhexadecyl, 2-butylhexadecyl, 2-hexylhexadecyl, 2-octylhexadecyl, n-heptadecyl, n-octadecyl, n-nonadecyl, n-eicosyl, 2-ethyleicosyl, 2-butyleicosyl, 2-octyleicosyl, n-heneicosyl, n-docosyl, n-tricosyl, n-tetracosanyl, n-pentacosanyl, n-hexacosanyl, n-heptacosanyl, n-octacosanyl, n-nonacosanyl, n-triacosanyl, adamantane, and the like.

As used herein, an aromatic group refers to a hydrocarbon group containing at least one aromatic ring. A heteroaromatic group refers to an aromatic hydrocarbon group containing at least one heteroatom. The heteroatom is preferably selected from Si, N, P, O, S and/or Ge, particularly preferably from Si, N, P, O and/or S. A fused ring aromatic group refers to a ring of an aromatic group which may have two or more rings, in which two carbon atoms are shared by two adjacent rings, i.e., a fused ring. A fused heterocyclic aromatic group refers to a fused ring aromatic hydrocarbon group containing at least one heteroatom. For purposes of the present application, an aromatic group or a heteroaromatic group includes not only aromatic ring systems, but also non-aromatic ring systems. Therefore, systems such as pyridine, thiophene, pyrrole, pyrazole, triazole, imidazole, oxazole, oxadiazole, thiazole, tetrazole, pyrazine, pyridazine, pyrimidine, triazine, carbene and the like are also considered aromatic or heterocyclic aromatic groups for the purposes of the invention. For purposes of the present application, a fused aromatic or fused heteroaromatic ring system includes not only systems of aromatic or heteroaromatic groups, but also a plurality of aromatic groups or heterocyclic aromatic groups may be interrupted by short non-aromatic units (<10% of non-H atoms, preferably less than 5% of non-H atoms, such as C, N or O atoms). Therefore, systems such as 9,9'-spirobifluorene, 9,9-diarylfluorene, triarylamines, diaryl ethers and the like are also considered to be fused aromatic ring systems for the purposes of this invention.

In some preferred embodiments, the aromatic group is selected from benzene, naphthalene, anthracene, fluoranthene, phenanthrene, benzophenanthrene, perylene, tetracene, pyrene, benzopyrene, acenaphthene, fluorene, and derivatives thereof; the heteroaromatic group is selected from triazine, pyridine, pyrimidine, imidazole, furan, thiophene, benzofuran, benzothiophene, indole, carbazole, pyrroloimidazole, pyrrolopyrrole, thienopyrrole, thienothiophene, furopyrrole, furofuran, thienofuran, benzisoxazole, benzisothiazole, benzimidazole, quinoline, isoquinoline, o-naphthyridine, quinoxaline, phenanthridine, primidine, quinazoline, quinazolinone, dibenzofuran, dibenzothiophene, carbazole and derivatives thereof.

In the present application, "*" indicates a linkage site or a fusion site.

In the present application, in a case that the linkage site is not specified in a group, it means that any linkage site in a group can be used as a linkage site.

In the present application, in a case that the fusion site is not specified in a group, it means that any fusion site in a group can be used as a fusion site, preferably two or more sites in the adjacent position of the group are fusion sites.

A non-aromatic ring refers to a ring system containing at least one non-aromatic ring. In the present application, preferably, the non-aromatic ring system only contains rings only formed by carbon-carbon single bonds.

In the present application, a single bond connected to a substituent extends through the corresponding ring, meaning that the substituent may be connected to any position of the ring, for example, R in 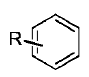 may be connected to any substitutable position of the phenyl ring.

In the present application, adjacent groups may form a ring with each other, which means that adjacent groups combine to form a substituted or unsubstituted aliphatic ring system, a substituted or unsubstituted aromatic ring system, a substituted or unsubstituted aliphatic heterocyclic ring system, or a substituted or unsubstituted heteroaromatic ring system. The above-mentioned ring system may be monocyclic or polycyclic.

The present application provides a heterocyclic group-containing organic compound, which has a structure represented by formula (1) or (2): 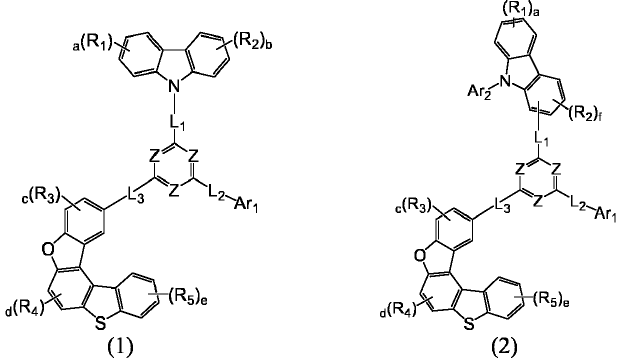 wherein:
Z is independently selected from CR₆ or an N atom, wherein at least one Z is an N atom;
R₁, R₂ or R₆ are each independently selected from -H, -D, or a linear alkyl group having 1 to 20 carbon atoms, a linear alkoxy group having 1 to 20 carbon atoms, or a linear thioalkoxy group having 1 to 20 carbon atoms, or a branched alkyl group having 3 to 20 carbon atoms, or a cyclic alkyl group having 3 to 20 carbon atoms, or a branched alkoxy group having 3 to 20 carbon atoms, or a cyclic alkoxy group having 3 to 20 carbon atoms, or a branched thioalkoxy group having 3 to 20 carbon atoms, or a cyclic thioalkoxy group having 3 to 20 carbon atoms, or a silyl group, or a ketone group having 1 to 20 carbon atoms, or an alkoxycarbonyl group having 2 to 20 carbon atoms, or an aryloxycarbonyl group having 7 to 20 carbon atoms, a cyano group, a carbamoyl group, a haloformyl group, a formyl group, an isocyano group, an isocyanate group, a thiocyanate group or an isothiocyanate group, a hydroxyl group, a nitro group, -CF₃, -Cl, -Br, -F, -I, or substituted or unsubstituted aromatic group having 6 to 60 ring atoms, or a substituted or unsubstituted heteroaromatic groups having 5 to 60 ring atoms, or a substituted or unsubstituted aryloxy group or heteroaryloxy group having 5 to 60 ring atoms, or a combinations thereof;
R₃, R₄ or R₅ are each independently selected from -H, -D, or a linear alkyl group having 1 to 20 carbon atoms, or a branched or cyclic alkyl group having 3 to 20 carbon atoms, or a phenyl group;
An is selected from a substituted or unsubstituted aromatic group having 6 to 30 ring atoms, or a substituted or unsubstituted heteroaromatic group having 5 to 30 ring atoms;
Ar₂ is selected from -H, -D, or a linear alkyl group having 1 to 20 carbon atoms, or a branched or cyclic alkyl group having 3 to 20 carbon atoms, or a substituted or unsubstituted aromatic group having 6 to 30 ring atoms, or a substituted or unsubstituted heteroaromatic group having 5 to 30 ring atoms;
L₁, L₂ and L₃ are each independently selected from a single bond, or a substituted or unsubstituted aromatic group having 6 to 30 ring atoms, or a substituted or unsubstituted heteroaromatic group having 5 to 30 ring atoms; and
a, b and e are each independently selected from 0, 1, 2, 3, or 4; d is selected from 0, 1 or 2; and c and f are each independently selected from 0, 1, 2 or 3.

In an embodiment, the substituent in the present invention is preferably selected from an alkyl group having 1 to 30 carbon atoms, an aromatic group having 6 to 20 ring atoms, or a heteroaromatic group having 5 to 20 ring atoms. Further, the substituent is selected from an alkyl group having 1 to 8 carbon atoms, an aromatic group having 6 to 10 ring atoms, or a heteroaromatic group having 6 to 13 ring atoms.

Optionally, the structure of the heterocyclic group-containing organic compound is selected from one of formulas (3-1) to (3-14):

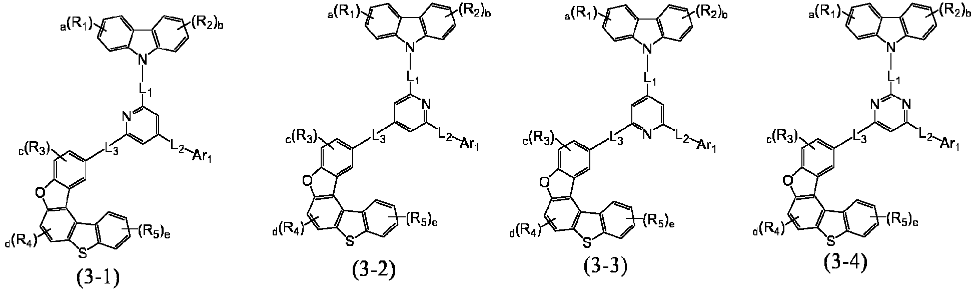

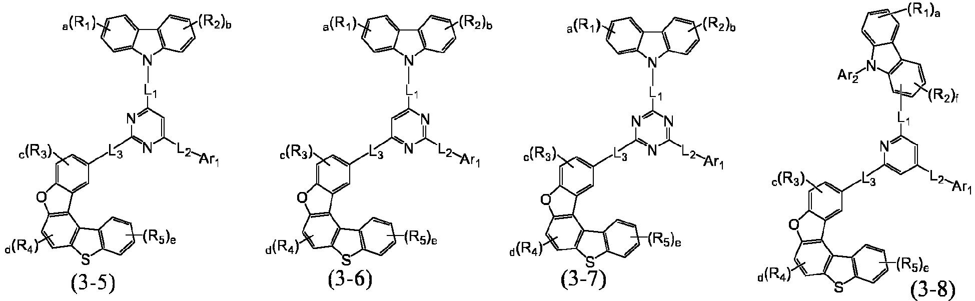

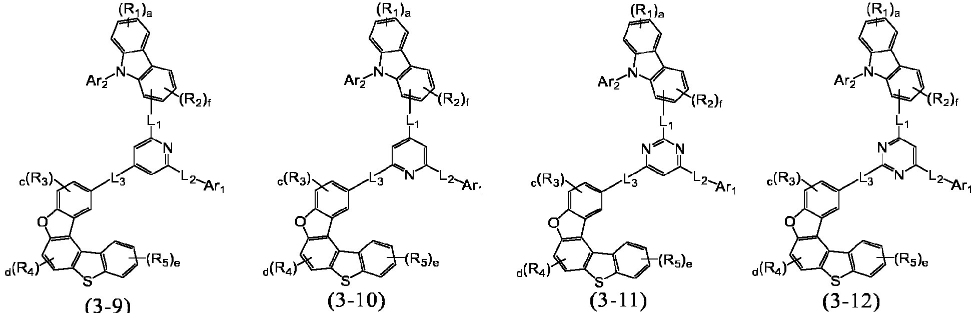

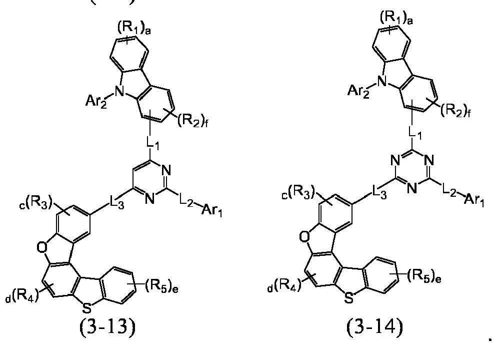

In an embodiment, L₁, L₂ and L₃ are each independently selected from a single bond, or a substituted or unsubstituted aromatic group having 6 to 20 ring atoms, or a substituted or unsubstituted heteroaromatic group having 6 to 20 ring atoms.

Further, L₁, L₂ and L₃ are each independently selected from a single bond, a substituted or unsubstituted phenyl, a substituted or unsubstituted biphenyl, a substituted or unsubstituted terphenyl, a substituted or unsubstituted pyridyl, a substituted or unsubstituted pyrimidinyl, a substituted or unsubstituted triazinyl, or a substituted or unsubstituted naphthyl. Further, the substituted substituent is selected from -D, or a linear alkyl group having 1 to 10 carbon atoms, or a branched or cyclic alkyl group having 3 to 10 carbon atoms, or a phenyl group, or a naphthyl group.

Further, L₁, L₂ and L₃ are each independently selected from a single bond or 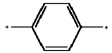 or 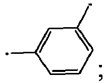 wherein: * indicates a linkage site.

In an embodiment, the structure of the heterocyclic group-containing organic compound is selected from one of formulas (4-1) to (4-8):

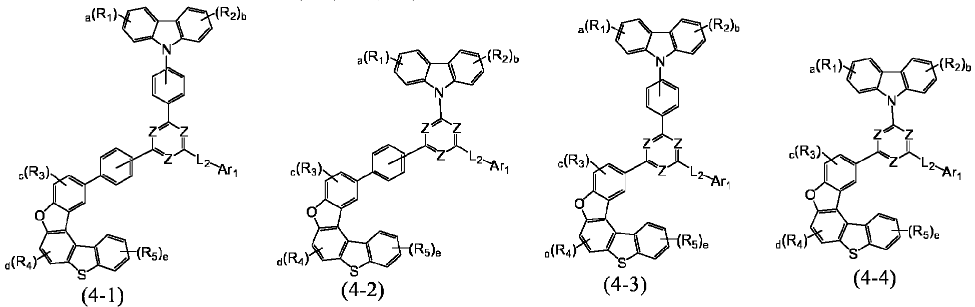

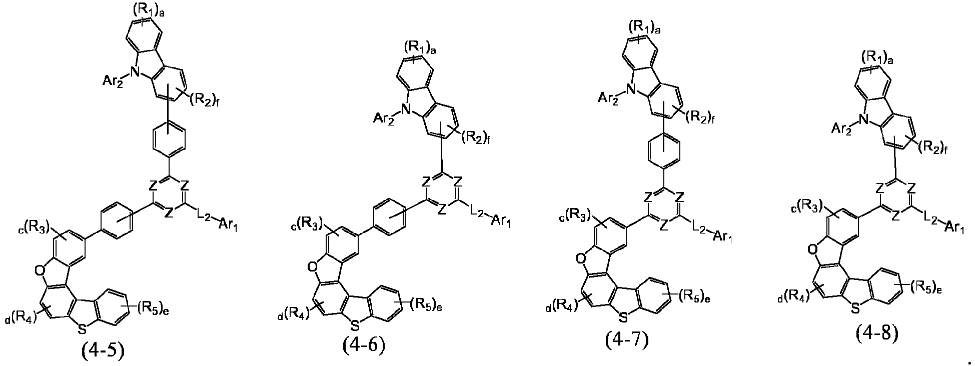

Preferably, Z in the formulas (4-1) to (4-8) is selected from N.

In an embodiment, R₁ or R₂ are each independently selected from -H, -D, or a linear alkyl group having 1 to 10 carbon atoms, or a branched or cyclic alkyl group having 3 to 10 carbon atoms, or a substituted or unsubstituted phenyl group, a substituted or unsubstituted biphenyl group, a substituted or unsubstituted terphenyl group, a substituted or unsubstituted naphthyl group, a substituted or unsubstituted phenanthryl group, a substituted or unsubstituted fluorenyl group, a substituted or unsubstituted dibenzofuranyl group, a substituted or unsubstituted dibenzothiophenyl group, a substituted or unsubstituted carbazolyl group. Further, the substituent is selected from -D, or a linear alkyl group having 1 to 10 carbon atoms, or a branched or cyclic alkyl group having 3 to 10 carbon atoms, or a phenyl group, or a naphthyl group.

In an embodiment, R₃, R₄ or R₅ are each independently selected from -H, -D, or a linear alkyl group having 1 to 10 carbon atoms, or a branched or cyclic alkyl group having 3 to 10 carbon atoms, or a phenyl group.

In an embodiment, c is selected from 0. Further, d is selected from 0.

In an embodiment, a, e and f are each independently selected from 0 or 1. Further, R₅ is selected from -H, -D, methyl, ethyl, n-propyl, isopropyl, n-butyl, sec-butyl, tert-butyl, isobutyl or phenyl.

In an embodiment, R₆ is independently selected from -H, -D, or a linear alkyl group having 1 to 10 carbon atoms, or a branched or cyclic alkyl group having 3 to 10 carbon atoms, or a substituted or unsubstituted phenyl group, a substituted or unsubstituted biphenyl group, a substituted or unsubstituted terphenyl group, a substituted or unsubstituted naphthyl group, a substituted or unsubstituted phenanthryl group, a substituted or unsubstituted fluorenyl group, a substituted or unsubstituted dibenzofuranyl group, a substituted or unsubstituted dibenzothiophenyl group, or a substituted or unsubstituted carbazolyl group. Further, the substituent is selected from -D, or a linear alkyl group having 1 to 10 carbon atoms, or a branched or cyclic alkyl group having 3 to 10 carbon atoms, or a phenyl group, or a naphthyl group.

In an embodiment, Ar₂ is selected from a linear alkyl group having 1 to 10 carbon atoms, or a branched or cyclic alkyl group having 3 to 10 carbon atoms, or a substituted or unsubstituted aromatic group having 6 to 10 ring atoms, or a substituted or unsubstituted heteroaromatic group having 5 to 10 ring atoms.

Further, Ar₂ is selected from methyl, ethyl, n-propyl, isopropyl, n-butyl, sec-butyl, tert-butyl, isobutyl, phenyl, or naphthyl, or a combination thereof.

In an embodiment, Ar₁ is selected from a substituted or unsubstituted aromatic group having 6 to 25 ring atoms, or a substituted or unsubstituted heteroaromatic group having 5 to 25 ring atoms.

Further, An is selected from one of following structures or a combination thereof: 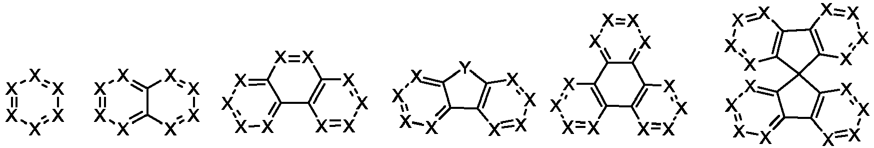 wherein:
X is independently selected from CR₇ or N;
Y is independently selected from C(R₈R₉), NR₈, Si(R₈R₉), O, S, Se, S=O, S(=O)₂, or PR₈;
R₇ to R₉ is independently selected from -H, -D, or a linear alkyl group having 1 to 20 carbon atoms, a linear alkoxy group having 1 to 20 carbon atoms, or a linear thioalkoxy group having 1 to 20 carbon atoms, or a branched or cyclic alkyl group having 3 to 20 carbon atoms, a branched or cyclic alkoxy group having 3 to 20 carbon atoms, or a branched or cyclic thioalkoxy group having 3 to 20 C atoms, or a silyl group, or a ketone group having 1 to 20 carbon atoms, or an alkoxycarbonyl group having 2 to 20 carbon atoms, or an aryloxycarbonyl group having 7 to 20 carbon atoms, a cyano group, a carbamoyl group, a haloformyl group, a formyl group, an isocyano group, an isocyanate group, a thiocyanate group or an isothiocyanate group, a hydroxyl group, a nitro group, -CF₃, -Cl, -Br, -F, -I, a crosslinkable group, or a substituted or unsubstituted aromatic or heteroaromatic group having 5 to 60 ring atoms, or an aryloxy or heteroaryloxy group having 5 to 60 ring atoms, or a combination thereof; and adjacent R₇ form a ring or not with each other.

In a case that X is a linkage site, X is selected from C.

Optionally, in some embodiments of the present application, X is independently selected from CR₇. Further, R₇ is independently selected from -H, -D, or a linear alkyl group having 1 to 10 carbon atoms, a branched alkyl group or a cyclic alkyl group having 3 to 10 carbon atoms, or a substituted or unsubstituted aromatic or heteroaromatic group having 5 to 10 ring atoms.

Optionally, in some embodiments of the present application, Ar₁ is independently selected from one of the following structures or a combination thereof: 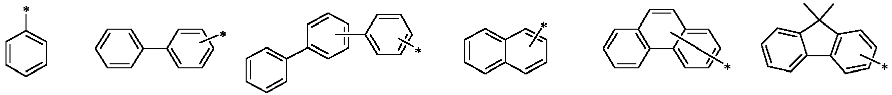 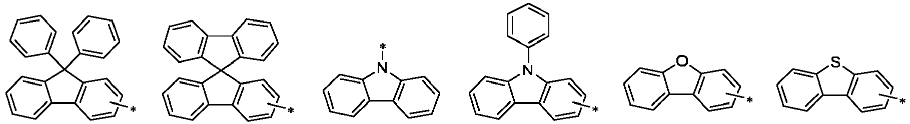 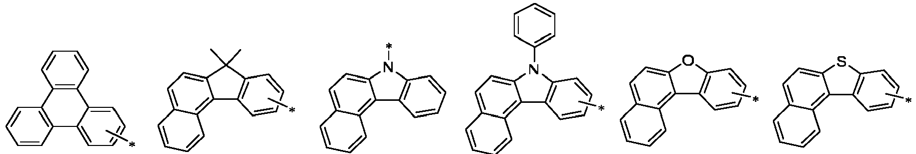 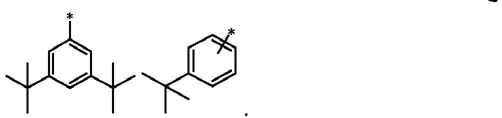 * is a linkage site.

Optionally, in some embodiments of the present application, the heterocyclic group-containing organic compound is preferably selected from, but not limited to, the following structures, which may be optionally substituted:

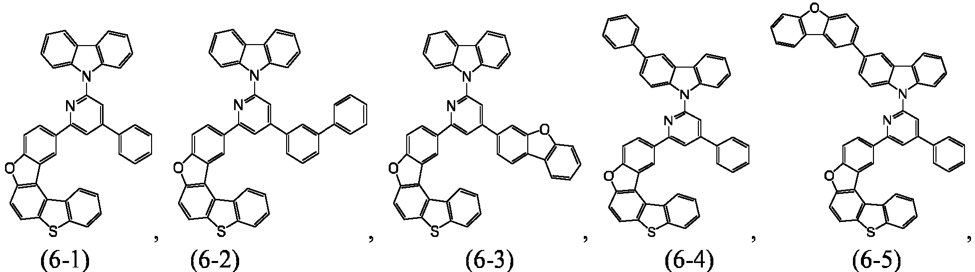

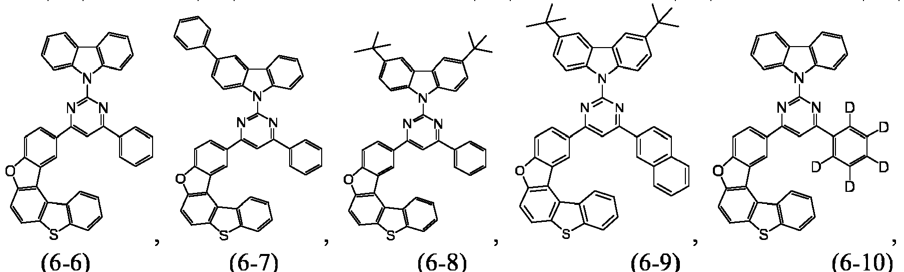

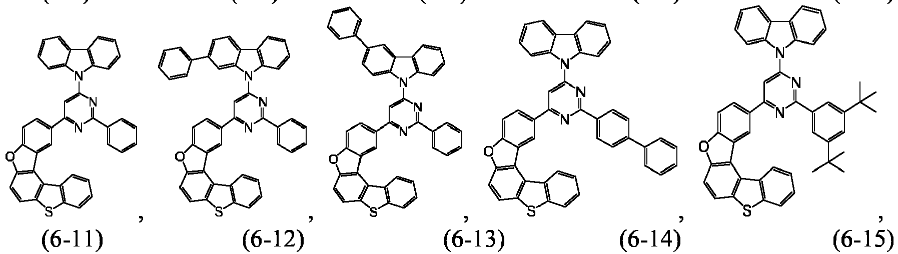

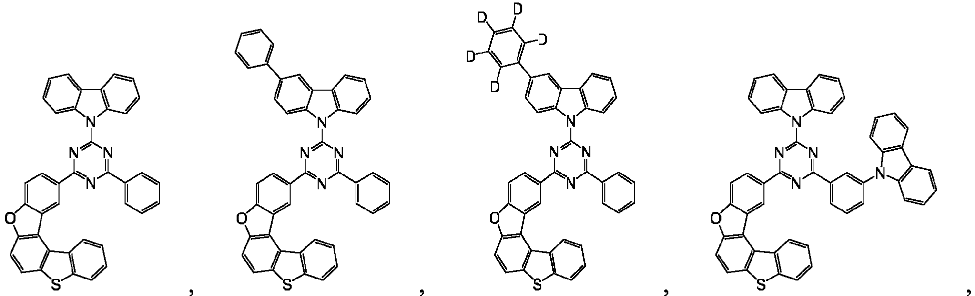

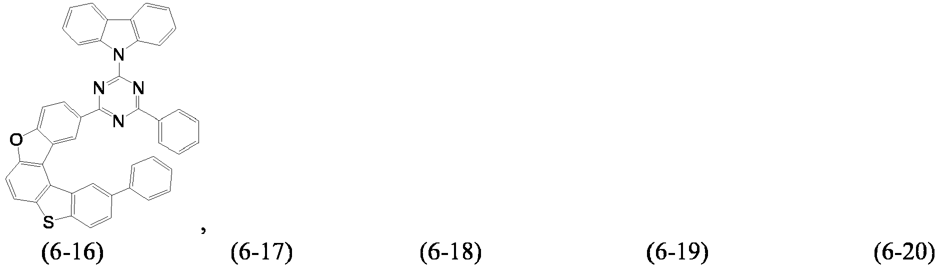

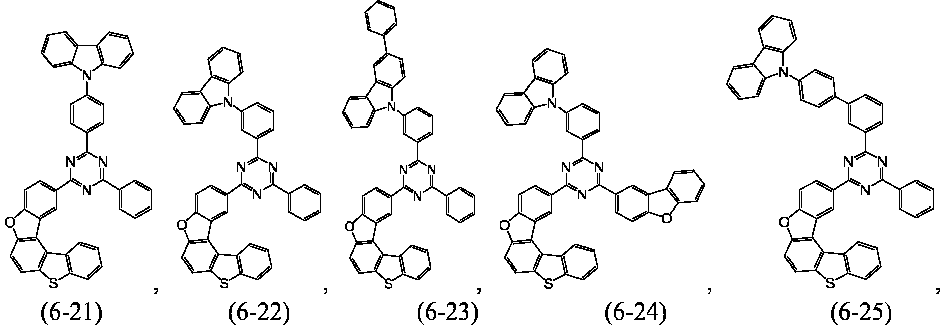

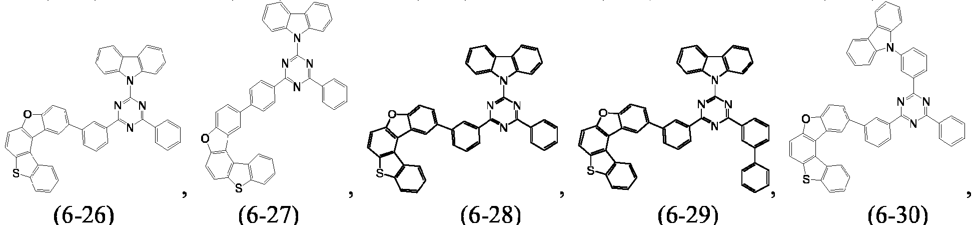

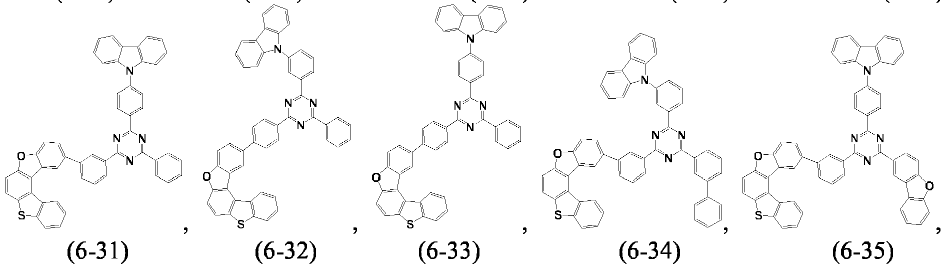

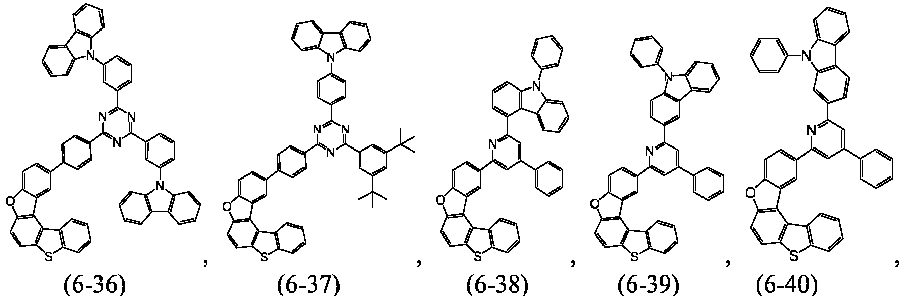

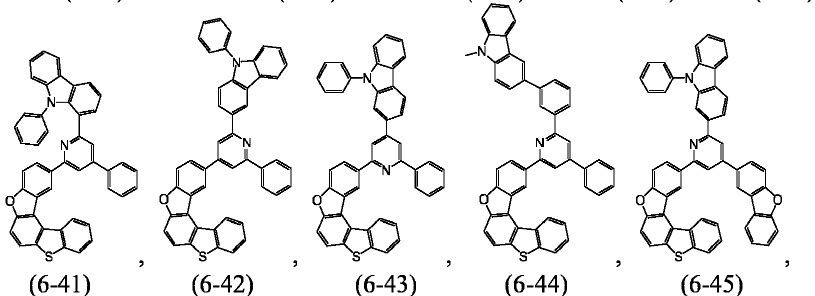

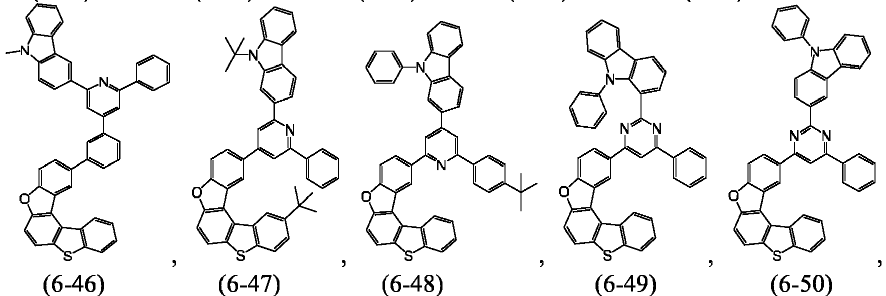

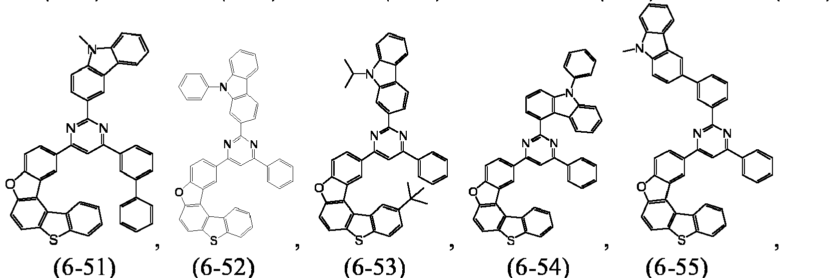

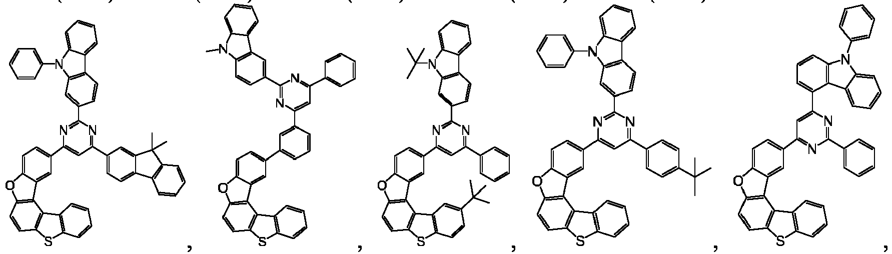

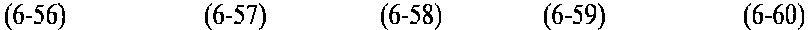

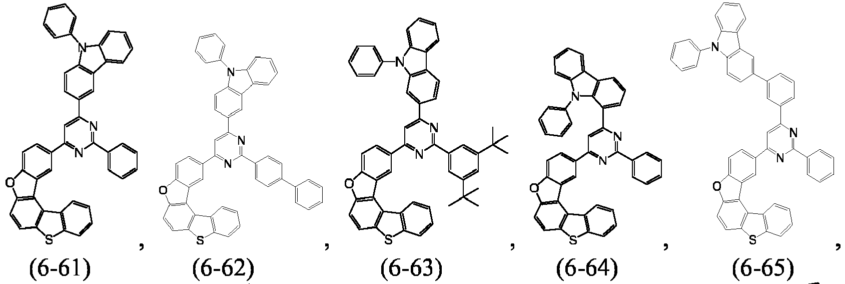

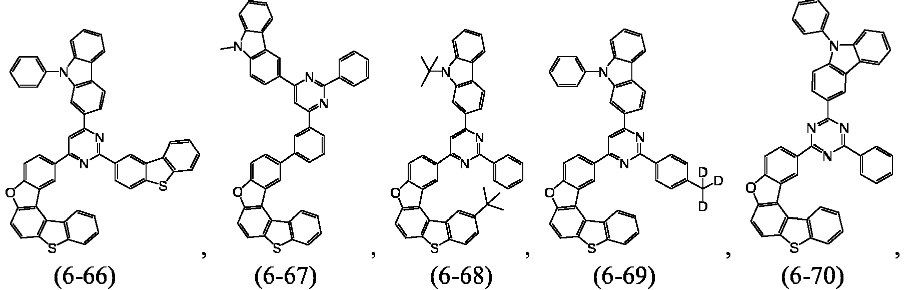

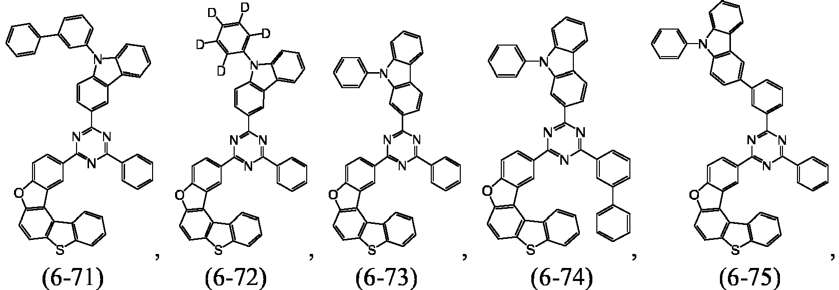

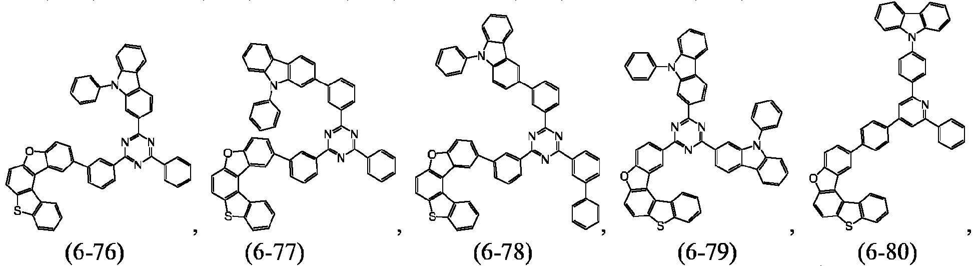

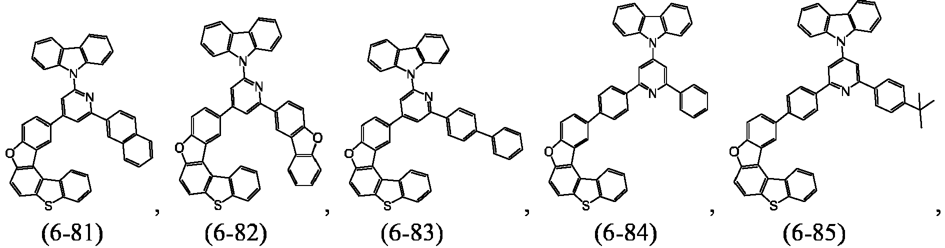

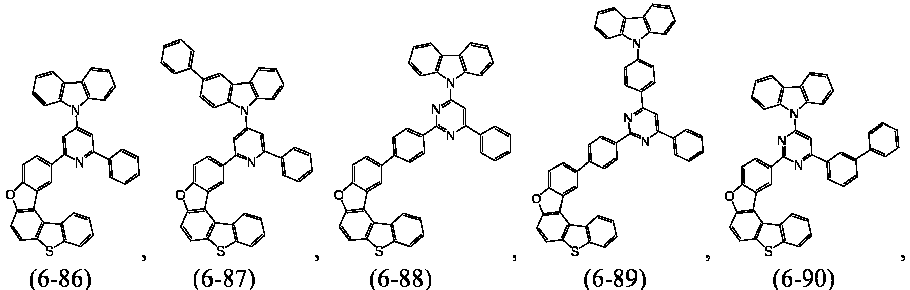

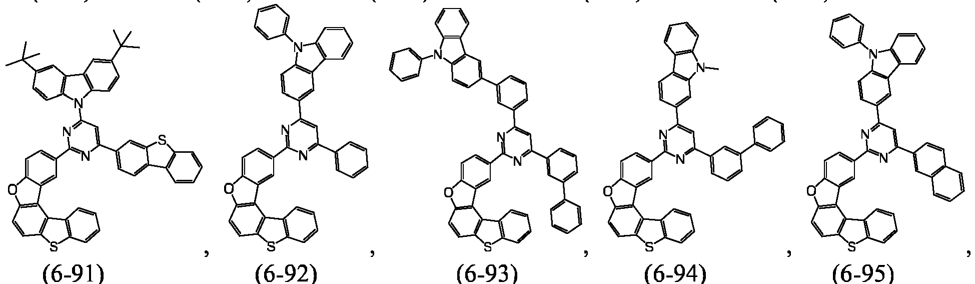

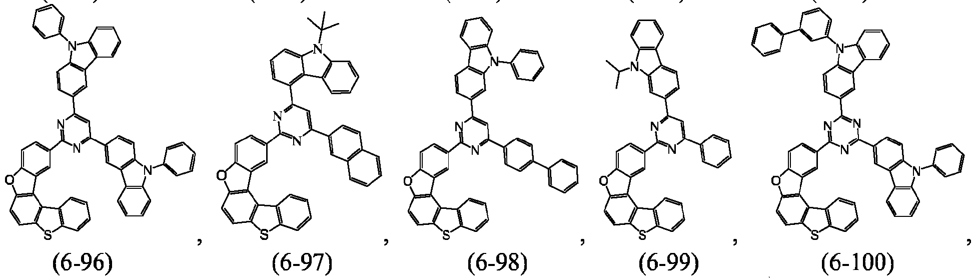

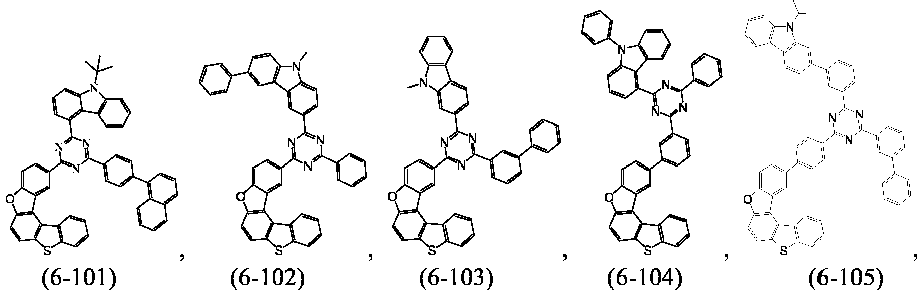

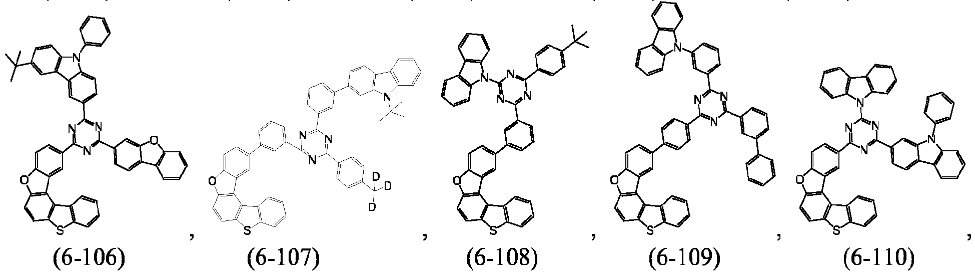

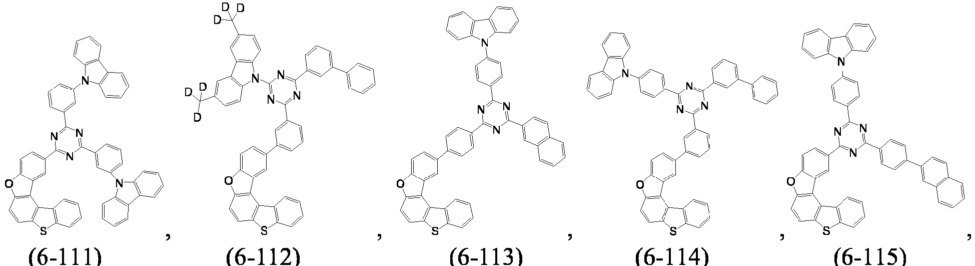

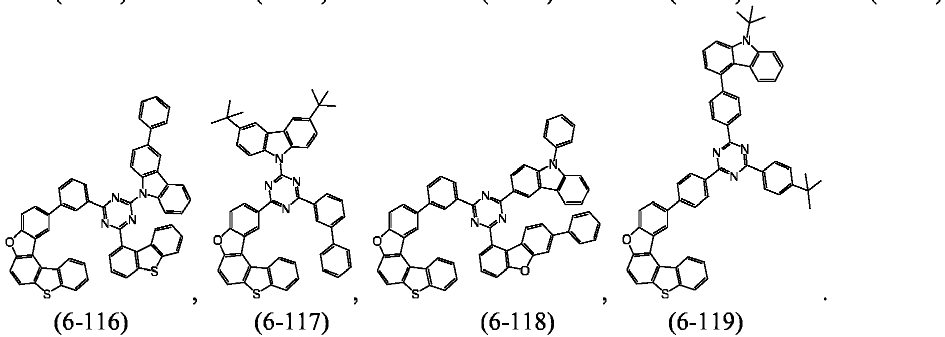

The heterocyclic group-containing organic compound according to the present invention can be used as a functional material in a functional layer of an electronic device. The organic functional layer includes, but is not limited to, a hole injection layer (HIL), a hole transport layer (HTL), an electron transport layer (ETL), an electron injection layer (EIL), an electron blocking layer (EBL), a hole blocking layer (HBL), and a light-emitting layer (EML).

In an embodiment, the heterocyclic group-containing organic compound according to the present invention is used in a light-emitting layer.

The present application further provides a mixture including at least one of the above-mentioned heterocyclic group-containing organic compounds and at least one organic functional material. The organic functional material is selected from a hole injection material, a hole transport material, an electron transport material, an electron injection material, an electron blocking material, a hole blocking material, a light-emitting material, a host material, or an organic dye. Various organic functional materials are described in detail, for example, in WO2010135519A1, US20090134784A1, and WO2011110277A1, and the entire contents of which are hereby incorporated herein by reference.

Optionally, in some embodiments of the present application, the organic functional material is selected from a hole-type transport material and is used as a co-host in electronic devices.

Optionally, in some embodiments of the present application, a molar ratio of the heterocyclic group-containing organic compound to the organic functional material ranges from 4:6 to 6:4.

In an embodiment, the molar ratio of the heterocyclic group-containing organic compound to the organic functional material is 5:5.

Optionally, in some embodiments of the present application, the organic functional material is represented by formulas (5-a) to (5-d): 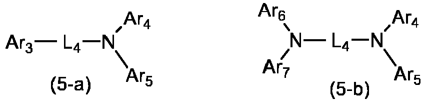 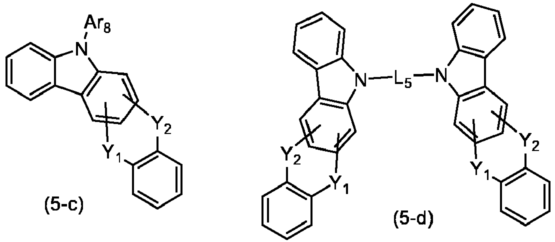 wherein,
Ar₃ to Ar₈ are each independently selected from a substituted or unsubstituted aromatic group having 6 to 30 ring atoms, or a substituted or unsubstituted heteroaromatic group having 5 to 30 ring atoms;
L₄ and L₅ are each independently selected from a substituted or unsubstituted aromatic group having 6 to 30 ring atoms, or a substituted or unsubstituted heteroaromatic group having 5 to 30 ring atoms;
Y₁ and Y₂ are each independently selected from C(R₁₀R₁₁), NR₁₀, O, S or a single bond; and;
R₁₀ to R₁₁ are each independently selected from -H, -D, or a linear alkyl group having 1 to 20 carbon atoms, or a branched or cyclic alkyl group having 3 to 20 carbon atoms, or a substituted or unsubstituted aromatic or heteroaromatic group having 5 to 60 ring atoms.

Any two of Ar₄, Ar₅, L₄ form a ring or not with each other.

Any two of Ar₆, Ar₇, L₄ form a ring or not with each other.

One or more H atoms in the formula may be substituted by one D atom or more D atoms.

Preferably, the above formed ring may be a 5-membered ring or a 6-membered ring.

Further, in an embodiment, the organic functional material is represented by one of formulas (5-f) to (5-i): 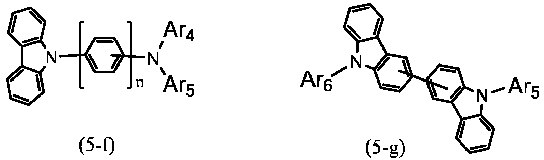 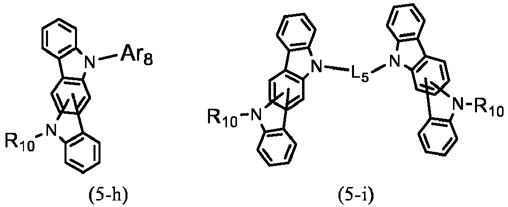 wherein: N is selected from 0 or 1 or 2 or 3 or 4; the H atom in the formula may be substituted by D.

Preferably, L₅ is selected from phenyl, biphenyl, terphenyl or naphthyl.

Preferably, Ar₄, Ar₅, Ar₆, Ar₈, R₁₀ are each independently selected from a substituted or unsubstituted aromatic group having 6 to 30 ring atoms or a substituted or unsubstituted heteroaromatic group having 5 to 30 ring atoms. Further, Ar₄, Ar₅, Ar₆, Ar₈, R₁₀ are each independently selected from a substituted or unsubstituted phenyl, a substituted or unsubstituted biphenyl, a substituted or unsubstituted terphenyl, a substituted or unsubstituted pyridyl, a substituted or unsubstituted pyrimidinyl, a substituted or unsubstituted triazinyl, a substituted or unsubstituted naphthyl, a substituted or unsubstituted phenanthryl, a substituted or unsubstituted triphenylene, a substituted or unsubstituted spirocyclyl, a substituted or unsubstituted dibenzofuranyl, a substituted or unsubstituted dibenzothienyl, a substituted or unsubstituted fluorenyl, or a substituted or unsubstituted carbazolyl. Further, the substituent is selected from -D, or a linear alkyl group having 1 to 10 carbon atoms, or a branched or cyclic alkyl group having 3 to 10 carbon atoms, or a phenyl group, or a biphenyl group, or a naphthyl group.

Preferably, the heterocyclic group-containing organic compound in the mixture is selected from formula (3-7) and the functional material is selected from formulas (5-f) or (5-g) or (5-i). Alternatively, the heterocyclic group-containing organic compound in the mixture is selected from formula (3-14), and the functional material is selected from formula (5-g) or (5-i).

Optionally, in some embodiments of the present application, the organic functional material is preferably selected from, but not limited to, the following structures:

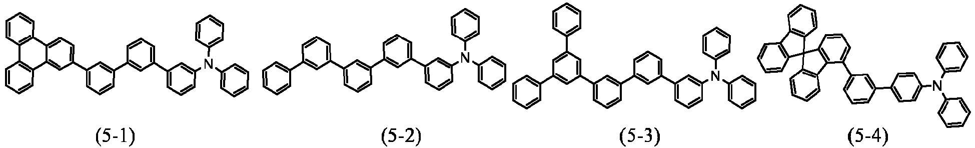

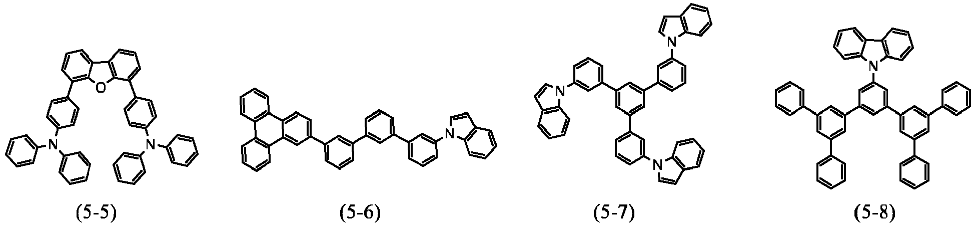

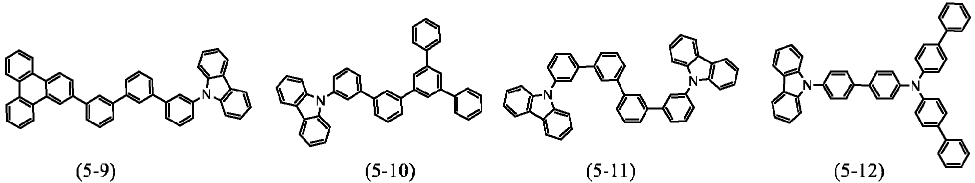

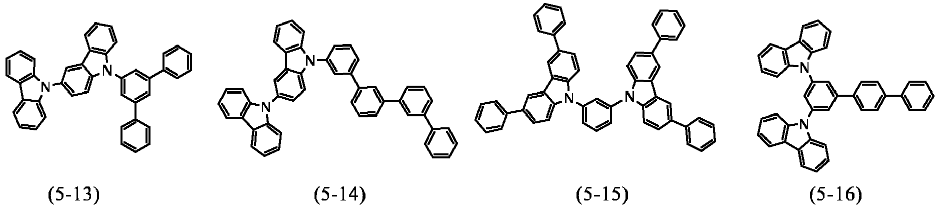

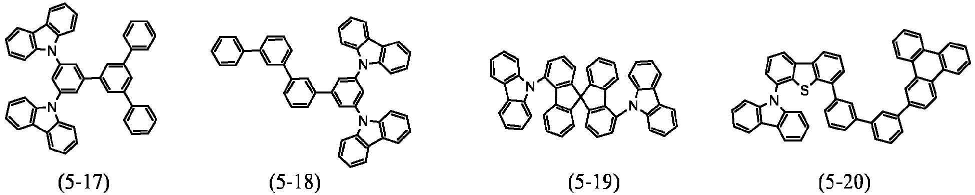

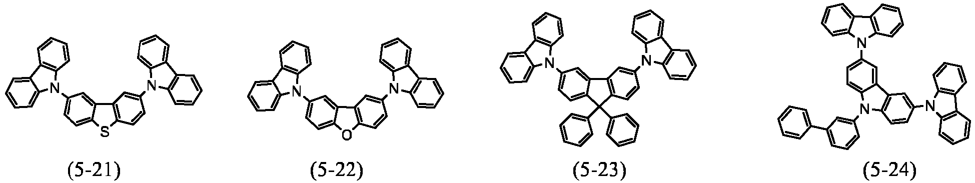

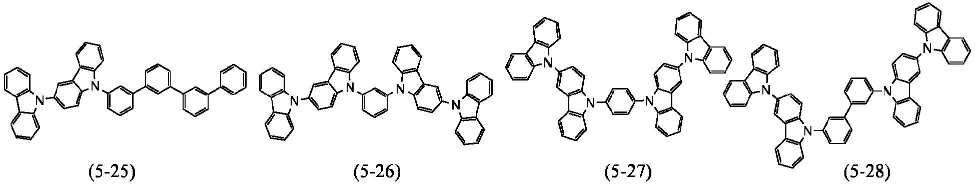

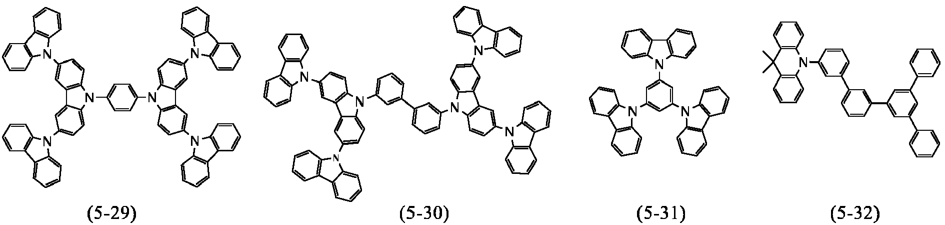

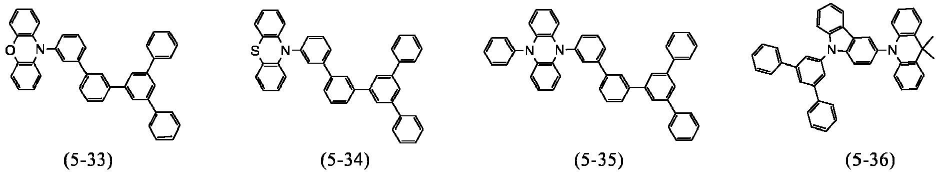

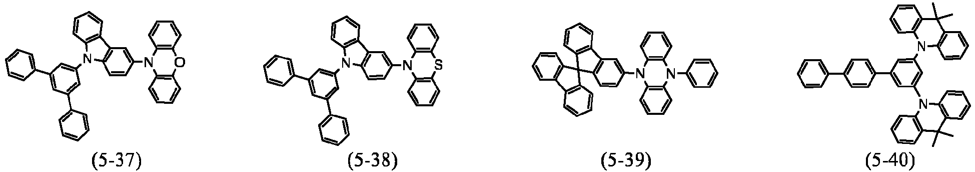

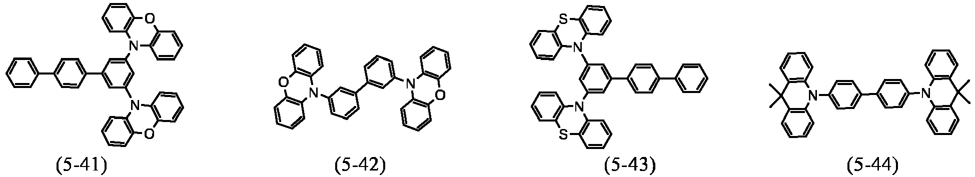

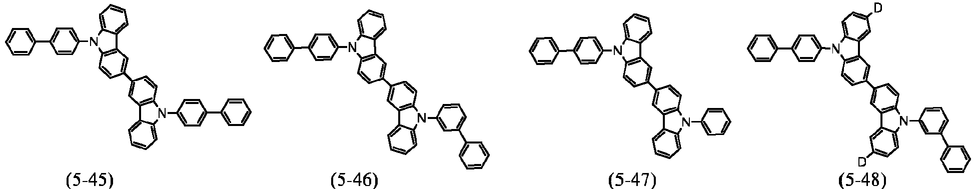

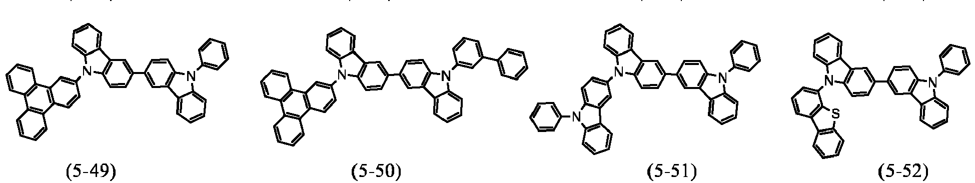

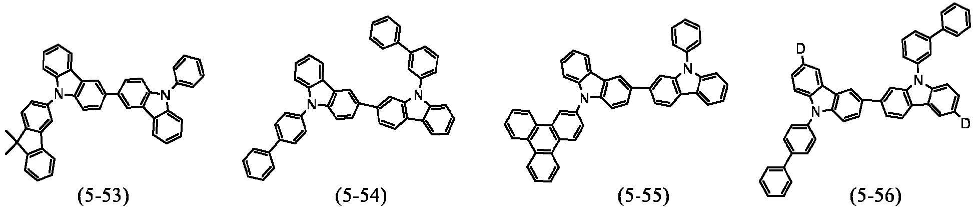

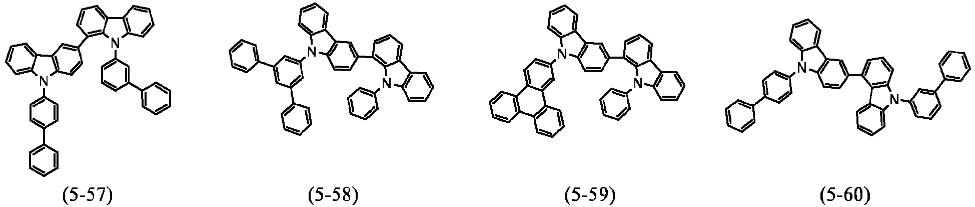

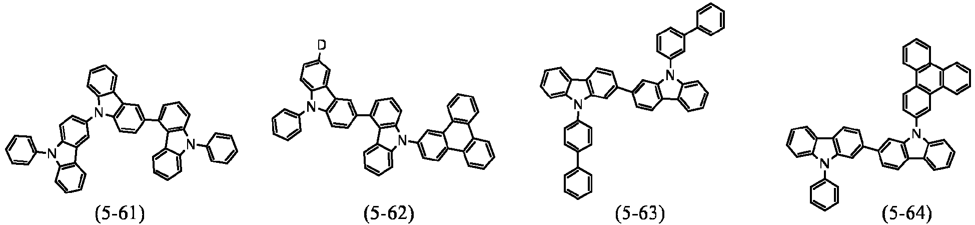

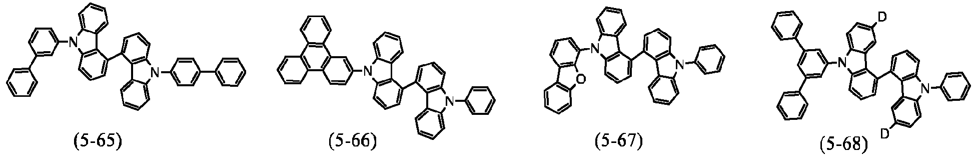

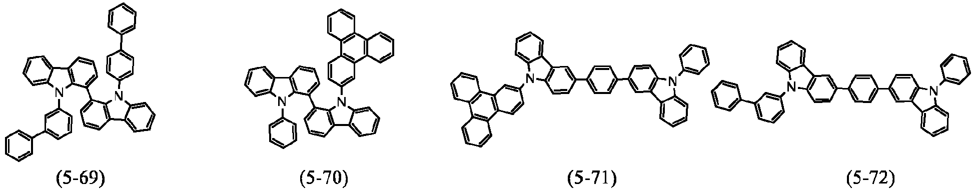

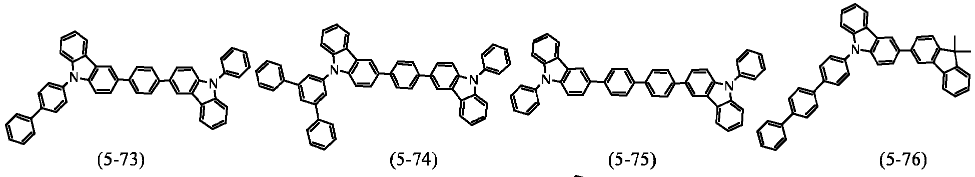

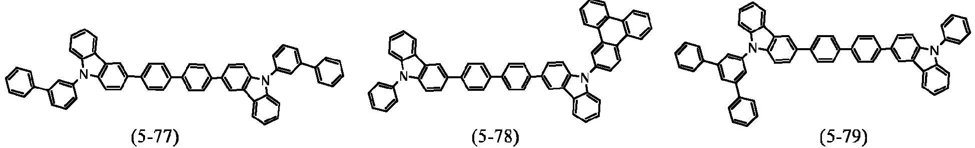

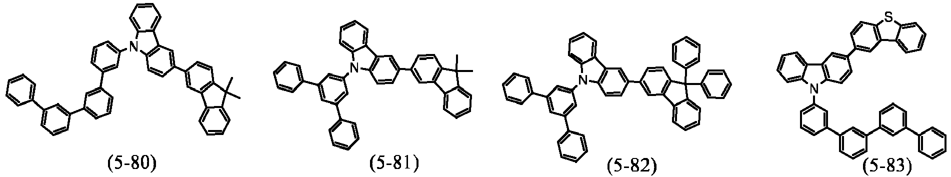

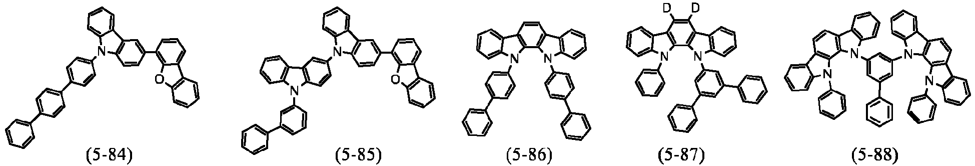

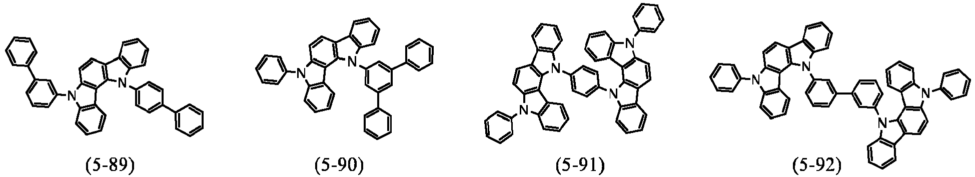

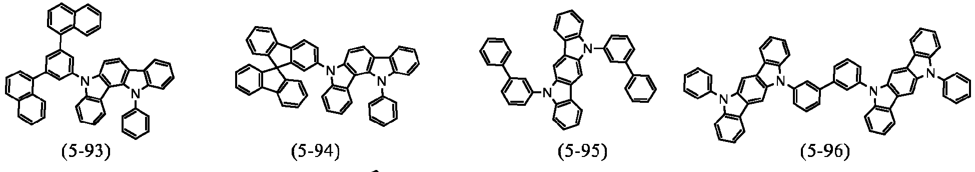

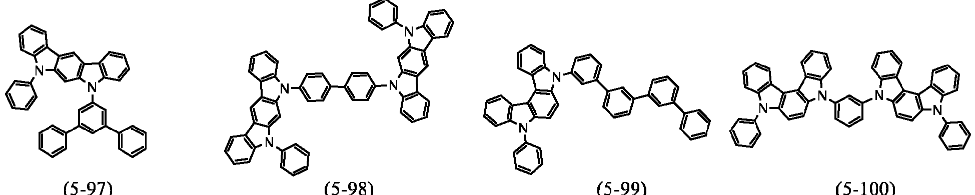

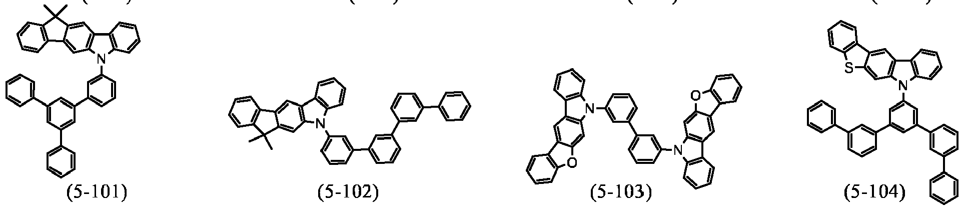

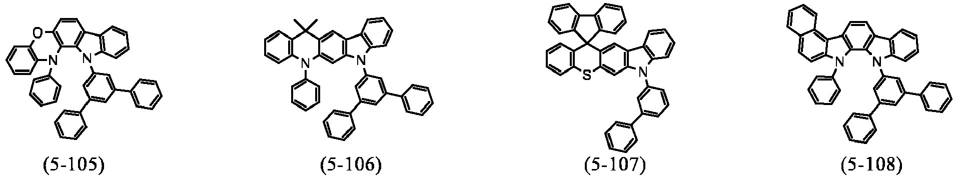

The present application further provides a composition including at least one of the above-mentioned heterocyclic group-containing organic compounds or a mixture thereof, and at least one organic solvent. The organic solvent is selected from at least one of aromatic or heteroaromatic group, an ester, an aromatic ketone or an aromatic ether, an aliphatic ketone or an aliphatic ether, a cycloaliphatic or an olefinic compound, a boronic ester or a phosphate ester compound.

Optionally, in some embodiments of the present application, the organic solvent is selected from an aromatic or a heteroaromatic-based solvent.

Examples of aromatic or heteroaromatic-based solvents suitable for the present application are, but are not limited to, p-diisopropylbenzene, pentylbenzene, tetrahydronaphthalene, cyclohexylbenzene, chloronaphthalene, 1, 4-dimethylnaphthalene, 3-isopropylbiphenyl, p-methylcumene, dipentylbenzene, tripentylbenzene, amyl-toluene, o-diethylbenzene, m-diethylbenzene, p-diethylbenzene, 1,2,3,4-tetramethylbenzene, 1,2,3,5-tetramethylbenzene, 1,2,4,5-tetramethylbenzene, butylbenzene, dodecylbenzene, dihexylbenzene, dibutylbenzene, p-diisopropylbenzene, cyclohexylbenzene, benzylbutylbenzene, dimethylnaphthalene, 3-isopropylbiphenyl, p-methylcumene, 1-methylnaphthalene, 1,2,4-trichlorobenzene, 4,4-difluorodiphenylmethane, 1,2-dimethoxy-4-(1-propenyl)benzene, diphenylmethane, 2-phenylpyridine, 3-phenylpyridine, N-methyldiphenylamine, 4-isopropylbiphenyl, α,α-dichlorodiphenylmethane, 4-(3-phenylpropyl)pyridine, benzyl benzoate, 1,1-bis(3,4-dimethylphenyl)ethane, 2-isopropylnaphthalene, quinoline, isoquinoline, methyl 2-furanoate, ethyl 2-furanoate, and the like.

Examples of aromatic ketone-based solvents suitable for the present application are, but are not limited to, 1-tetralone, 2-tetralone, 2-(phenylepoxy) tetralone, 6-(methoxy) tetralone, acetophenone, propiophenone, benzophenone, and derivatives thereof, such as 4-methylacetophenone, 3-methylacetophenone, 2-methylacetophenone, 4-methylacetophenone, 3-methylacetophenone, 2-methylacetophenone, and the like.

Examples of aromatic ether-based solvents suitable for the present application are, but are not limited to, 3-phenoxytoluene, butoxybenzene, p-anisaldehyde dimethyl acetal, tetrahydro-2-phenoxy-2H-pyran, 1,2-dimethoxy-4-(1-propenyl)benzene, 1,4-benzodioxane, 1,3-dipropylbenzene, 2,5-dimethoxytoluene, 4-ethylbenzoethyl ether, 1,3-dipropoxybenzene, 1,2,4-trimethoxybenzene, 4-(1-propenyl)-1,2-dimethoxybenzene, 1,3-dimethoxybenzene, glycidyl phenyl ether, dibenzyl ether, 4-tert-butyl anisole, trans-p-propenyl anisole, 1,2-dimethoxybenzene, 1-methoxynaphthalene, diphenyl ether, 2-phenoxymethyl ether, 2-phenoxytetrahydrofuran, ethyl-2-naphthyl ether, and the like.

Examples of aliphatic ketone-based solvents suitable for the present application are, but are not limited to, 2-nonanone, 3-nonanone, 5-nonanone, 2-decanone, 2,5-hexanedione, 2,6,8-trimethyl-4-nonanone, fenchone, phorone, isophorone, di-n-amyl ketone, and the like; or aliphatic ethers, such as amyl ether, hexyl ether, dioctyl ether, ethylene glycol dibutyl ether, diethylene glycol diethyl ether, diethylene glycol butyl methyl ether, diethylene glycol dibutyl ether, triethylene glycol dimethyl ether, triethylene glycol ethyl methyl ether, triethylene glycol butyl methyl ether, tripropylene glycol dimethyl ether, tetraethylene glycol dimethyl ether, and the like.

Examples of ester-based solvents suitable for the present application are, but are not limited to, alkyl octanoate, alkyl sebacate, alkyl stearate, alkyl benzoate, alkyl phenylacetate, alkyl cinnamate, alkyl oxalate, alkyl maleate, alkyl lactone, alkyl oleate, and the like. Octyl octanoate, diethyl sebacate, diallyl phthalate, isononyl isononanoate, and the like are particularly preferred.

Optionally, in some embodiments of the present application, the composition further includes a second organic solvent. The second organic solvent is selected from at least one of methanol, ethanol, 2-methoxyethanol, dichloromethane, trichloromethane, chlorobenzene, o-dichlorobenzene, tetrahydrofuran, anisole, morpholine, toluene, o-xylene, m-xylene, p-xylene, 1, 4-dioxane, acetone, methyl ethyl ketone, 1,2-dichloroethane, 3-phenoxytoluene, 1,1,1 -trichloroethane, 1,1,2,2-tetrachloroethane, ethyl acetate, butyl acetate, dimethylformamide, dimethylacetamide, dimethylsulfoxide, tetrahydronaphthalene, decalin, and indene.

Optionally, in some embodiments of the present application, a solvent particularly suitable for the present application is a solvent having a Hansen solubility parameter in the following ranges:
δd (dispersion force): ranges from 17.0 MPa^{1/2} to 23.2 MPa^{1/2}, especially ranges from 18.5 MPa^{1/2} to 21.0 MPa^{1/2};
Δp (polarity force) ranges from 0.2 MPa^{1/2} to 12.5 MPa^{1/2}, especially ranges from 2.0 MPa^{1/2} to 6.0 MPa^{1/2};
Δh (hydrogen bonding force) ranges from 0.9 MPa^{1/2} to 14.2 MPa^{1/2}, especially ranges from 2.0 MPa^{1/2} to 6.0 MPa^{1/2}.

For the compositions in the present application, the boiling point should be considered when selecting an organic solvent. In the present application, the boiling point of the organic solvent is greater than or equal to according to the present application °C; preferably greater than or equal to preferably °C; more preferably greater than or equal to more preferably °C; more preferably greater than or equal to more preferably °C; and most preferably greater than or equal to most preferably °C or greater than or equal to preferably °C. Boiling points in these ranges are beneficial to prevent nozzle clogging of inkjet print heads. The organic solvent can be evaporated from that solvent system to form a thin film containing functional materials.

Optionally, in some embodiments of the present application, the composition is a solution.

Optionally, in some embodiments of the present application, the composition is a suspension.

Optionally, in some embodiments of the present application, the heterocyclic group-containing organic compound may range from 0.01wt% to 15wt%, 0.1wt% to 10 wt%, 0.2wt% to 5 wt%, or 0.25 wt% to 3 wt%, based on the total mass of the composition.

The present application further provides an application of the composition as coating or printing ink in preparing organic electronic devices, particularly preferably by printing or coating.

Suitable printing or coating techniques include, but are not limited to, printing or coating processes include inkjet printing, nozzle printing, gravure printing, letterpress printing, screen printing, dip coating, spin coating, doctor blade coating, roller printing, twist roller printing, flexographic lithographic printing, flexographic printing, rotary printing, spraying, brushing or pad printing, slot-type extrusion coating, and the like, wherein gravure printing, jet printing and inkjet are preferable.

Optionally, in some embodiments of the present application, the solution or suspension may additionally include one or more components such as surface active compounds, lubricants, wetting agents, dispersing agents, hydrophobic agents, adhesives, and the like, for adjusting viscosity, film-forming performance, for adjusting viscosity, film-forming performance, improving adhesion, and the like.

The present application further provides applications of the above-mentioned heterocyclic group-containing organic compounds, the above-mentioned mixtures or the above-mentioned composition in organic electronic devices.

The present application further provides an organic electronic device. The organic electronic device includes a first electrode, a second electrode, and at least one organic functional layer disposed between the first electrode and the second electrode. The organic functional layer includes at least one of the above-mentioned heterocyclic group-containing organic compounds, the above-mentioned mixtures or is prepared from the above-mentioned compositions. Further, the organic electronic device includes a cathode, an anode, and one or more organic functional layers disposed between the cathode and the anode.

Optionally, in some embodiments of the present application, the organic functional layer may be selected from a hole injection layer, a hole transport layer, a light-emitting layer, an electron blocking layer, an electron injection layer, an electron transport layer, and a hole blocking layer.

Optionally, in some embodiments of the present application, the organic functional layer includes a light-emitting layer including at least one of the above-mentioned heterocyclic group-containing organic compounds, the above-mentioned mixtures or the above-mentioned compositions.

In an embodiment, the organic electronic device includes a light-emitting layer, wherein the light-emitting layer host material is selected from the above-mentioned heterocyclic group-containing organic compound, the above-mentioned mixture or the above-mentioned composition.

Optionally, in some embodiments of the present application, the organic electronic device may be selected from, but is not limited to, an organic light-emitting diode (OLED), an organic photovoltaic cell (OPV), an organic light-emitting cell (OLEEC), an organic field-effect transistor (OFET), an organic light-emitting field-effect transistor (OLED), an organic laser, an organic spin-electron device, an organic sensor, an organic plasma-emitting diode, and the like. Particularly preferred are organic electroluminescent devices, such as OLED, OLEEC, an organic light-emitting field-effect transistor.

The present application further provides an application of the above organic electronic device in an electronic device, including, but not limited to, a display device, an illumination device, a light source, a sensor, and the like.

The present application will be specifically described by way of specific examples, which are only part of the present application and are not intended to limit the present application.

### Specific examples

### Example 1

### Synthetic route of compound (6-4):

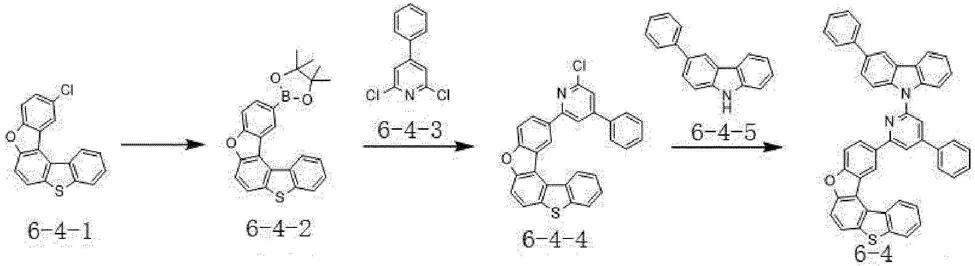

1) Synthesis of intermediate 6-4-2: compound 6-4-1 (15.5 g, 50 mmol), bis(pinacolato)diboron (12.7 g, 50 mmol), potassium acetate (9.8 g, 100 mmol) and [1,1'-bis (diphenylphosphino) ferrocene] dichloro palladium (PdCl₂(dppf)) (2.2 g, 3 mmol), together with 100 mL of 1,4-dioxane as a solvent were added to a three-necked flask of 250 mL under nitrogen atmosphere, which were heated under a temperature of 110 °C. The reaction was carried out for 12 hours to obtain a reaction solution. After the reaction was completed, the reaction solution was cooled to room temperature. The reaction solution was sucting filtered to collect the filtrate, followed by rotary evaporation to remove most of the solvent. Then washed with dichloromethane for 3 times to collect the organic solution. After rotary evaporation to dryness, recrystallization was carried out to obtain the intermediate 6-4-2 with a yield of 82%.
2) Synthesis of intermediate 6-4-4: compound 6-4-2 (12 g, 30 mmol), compound 6-4-3 (6.7 g, 30 mmol), tetrakis (triphenylphosphine) palladium (1.72 g, 1.5 mmol), tetrabutylammonium bromide (1.3 g, 4 mmol), sodium hydroxide (2 g, 50 mmol), water (20 mL) and toluene (150 mL) were added to a three-necked flask of 250 mL under nitrogen atmosphere, which were heated and stirred under a temperature of 80 °C for 12 hours. After the reaction was completed, a reaction solution was obtained, and most of the reaction solution was evaporated by rotary evaporation. Then the reaction solution was washed three times with dichloromethane to collect the organic solution. After rotary evaporation to dryness, recrystallization was carried out to obtain the intermediate 6-4-4 with a yield of 71%.
3) Synthesis of compound 6-4: compound 6-4-5 (4.86 g, 20 mmol), sodium hydride (0.96 g, 40 mmol), and 100 mL of dimethylformamide were added to a three-necked flask of 300 mL under nitrogen atmosphere, which were heated and stirred at room temperature for 2 hours. After that, 6-4-4 (9.24 g, 20 mmol) was further added to the three-necked flask at one time, and the reaction was continued for 12 hours under stirring. After the reaction was completed, a reaction solution was obtained. The reaction solution was poured into 300 mL pure water, and subjected to sucting filtering to collect filter residue. The filter residue was washed with water and methanol successively, then the filter residue was collected and purified by recrystallization to obtain compound 6-4 with a yield of 68%. MS (ASAP): 669.

### Example 2

### Synthesis route of compound (6-9):

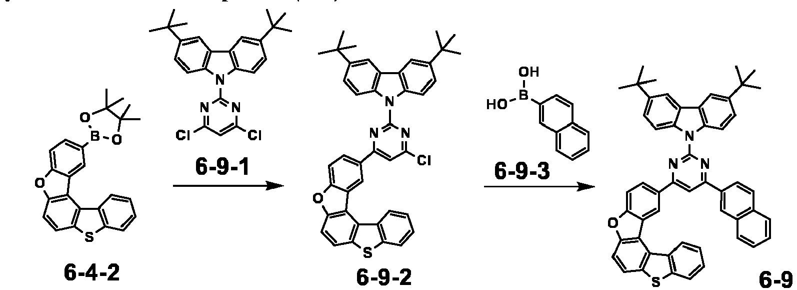

1) Synthesis of intermediate 6-9-2: the method is the same as the synthesis method of compound 6-4-4, except that compound 6-4-3 was replaced by compound 6-9-1, and the yield was 73%.
2) Synthesis of Compounds 6-9: the method is the same as the synthesis method of compound 6-4-4, except that compounds 6-4-2 and 6-4-3 were replaced by compounds 6-9-3 and 6-9-2, respectively, and the yield was 78%. MS (ASAP): 756.

### Example 3

### Synthesis route of compound (6-10):

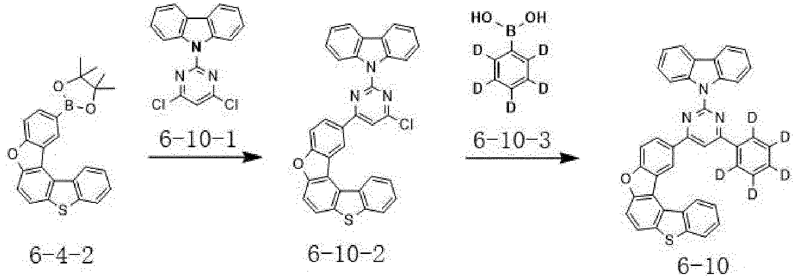

1) Synthesis of intermediate 6-10-2: the method is the same as the synthesis method of compound 6-4-4, except that compound 6-4-3 was replaced by compound 6-10-1, and the yield was 70%.
2) Synthesis of compounds 6-10: the method is the same as the synthesis method of compound 6-4-4, except that compounds 6-4-2 and 6-4-3 were replaced by compounds 6-10-3 and 6-10-2, respectively, and the yield was 75%. MS (ASAP): 599.

### Example 4

### Synthetic route of compound (6-12):

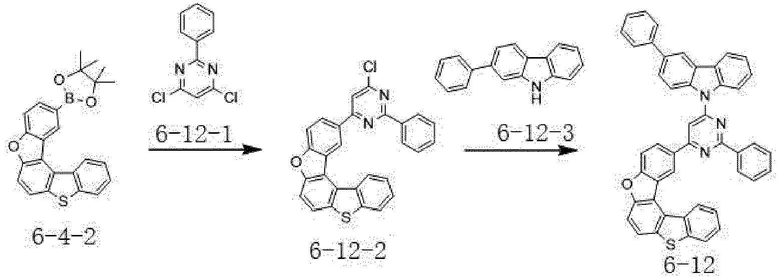

1) Synthesis of intermediate 6-12-2: the method is the same as the synthesis method of compound 6-4-4, except that compound 6-4-3 was replaced by compound 6-12-1, and the yield was 73%.

Synthesis of compound 6-12: the method is the same as the synthesis method of compound 6-4-4, except that compounds 6-4-4 and 6-4-5 were replaced by compounds 6-12-2 and 6-12-3, respectively, and the yield was 72%. MS (ASAP): 670.

### Example 5

### Synthetic route of compound (6-16):

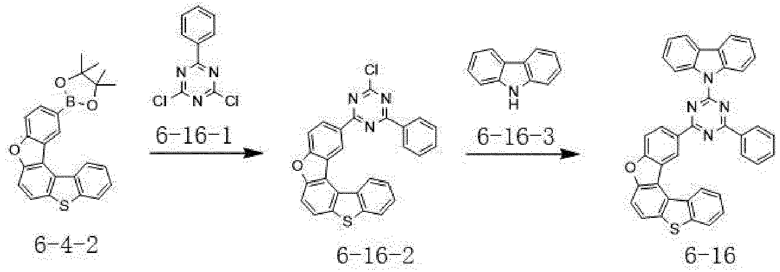

1) Synthesis of intermediate 6-16-2: the method is the same as the synthesis method of compound 6-4-4, except that compound 6-4-3 was replaced by compound 6-16-1, and the yield was 68%.
2) Synthesis of compound 6-16: the method is the same as the synthesis method of compound 6-4, except that compounds 6-4-4 and 6-4-5 were replaced by compounds 6-16-2 and 6-16-3, respectively, and the yield was 74%. MS (ASAP): 595.

### Example 6

### Synthesis route of compound (6-17):

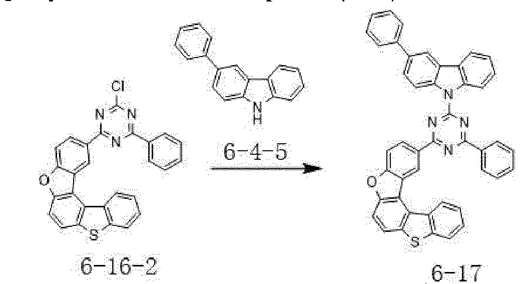

1) Synthesis of compound 6-17: the method is the same as the synthesis method of compound 6-4, except that compound 6-4-4 was were replaced by compound 6-16-2, and the yield was 75%. MS (ASAP): 671.

### Example 7

### Synthetic route of compound (6-20):

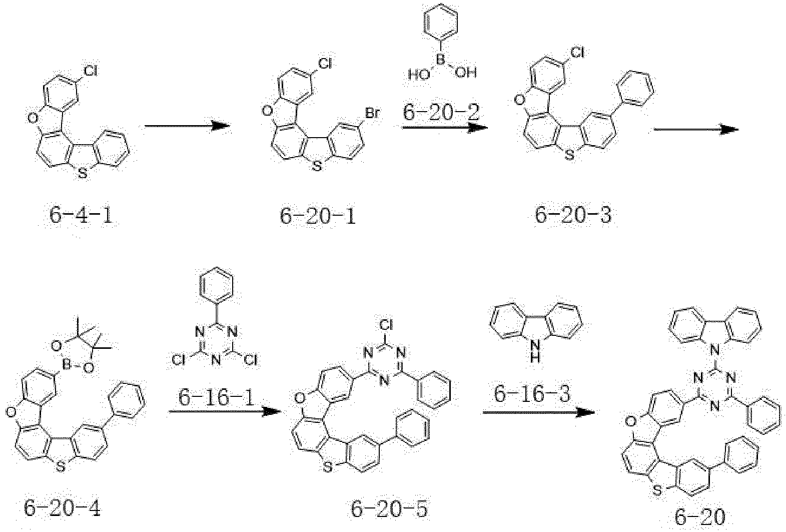

1) Synthesis of intermediate 6-20-1: compound 6-4-1 (30.9 g, 100 mmol), iron powder (0.28 g, 5 mmol) and trichloromethane (150 mL) were added to a three-necked flask of 300 mL, stirred and dissolved, then 100 mmol bromine water was slowly added dropwise in an ice bath. After the addition was completed, the reaction temperature was allowed to rise to room temperature naturally, and the reaction was continued for 6 hours under stirring. After the reaction was completed, the reaction was quenched by adding aqueous ammonium chloride solution. The reaction solution was extracted with dichloromethane and washed with water for three times to collect the organic solution. After rotary evaporation to dryness, recrystallization was carried out to obtain the intermediate 6-20-1 with a yield of 82%.
2) Synthesis of intermediate 6-20-3: the method is the same as the synthesis method of compound 6-4-4, except that compounds 6-4-2 and 6-4-3 were replaced by compounds 6-20-2 and 6-20-1 with a yield of 80%.
3) Synthesis of intermediate 6-20-4: the method is the same as the synthesis method of compound 6-4-2, except that compound 6-4-1 was replaced by compound 6-20-3 with a yield of 84%.
4) Synthesis of intermediate 6-20-5: the method is the same as the synthesis method of compound 6-4-4, except that compounds 6-4-3 and 6-4-4 were replaced by compounds 6-20-4 and 6-16-1 with a yield of 72%.
5) Synthesis of Compound 6-20: the method is the same as the synthesis method of compound 6-4, except that compounds 6-4-4 and 6-4-5 were replaced by compounds 6-20-5 and 6-16-3, respectively, and the yield was 75%. MS (ASAP): 671.

### Example 8

### Synthesis route of compound (6-22):

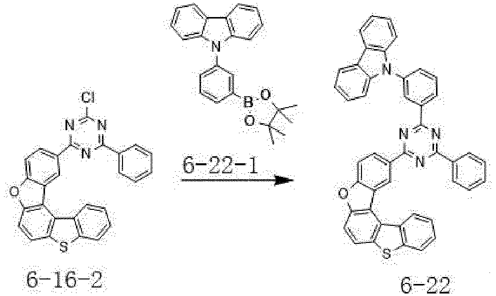

1) Synthesis of compound 6-22: the method is the same as the synthesis method of compound 6-4-4, except that compounds 6-4-2 and 6-4-3 were replaced by compounds 6-22-1 and 6-16-2, respectively, and the yield was 75%. MS (ASAP): 671.

### Example 9

### Synthesis route of compound (6-24):

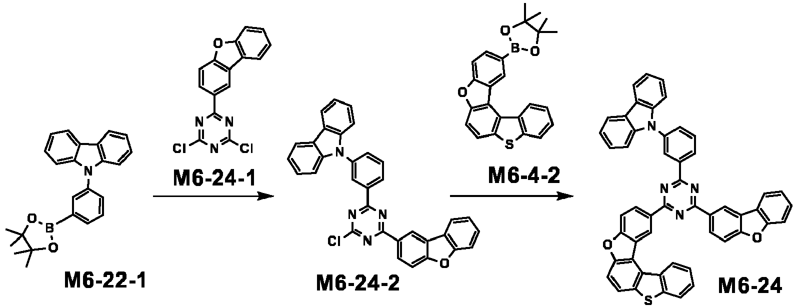

Synthesis of intermediate 6-24-2: the method is the same as the synthesis method of compound 6-4-4, except that compounds 6-4-2 and 6-4-3 were replaced by compounds 6-22-1 and 6-24-1 with a yield of 73%.

Synthesis of Compound 6-24: the method is the same as the synthesis method of compound 6-4-4, except that compounds 6-4-2 and 6-4-3 were replaced by compounds 6-4-2 and 6-24-2, respectively, and the yield was 82%. MS (ASAP): 761.

### Example 10

### Synthetic route of compound (6-26):

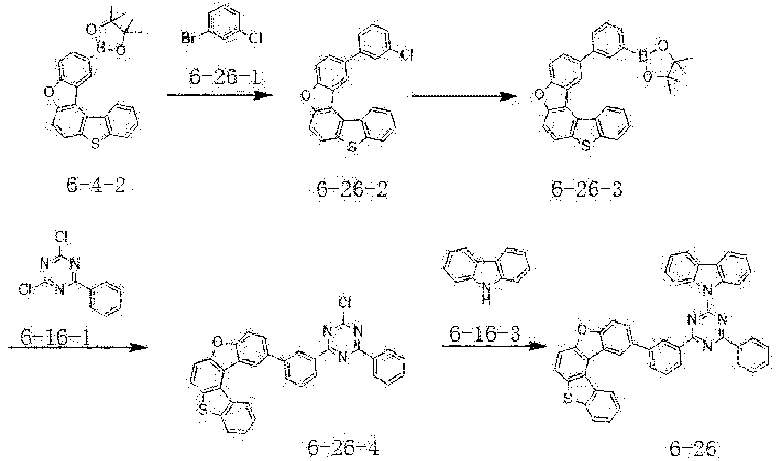

1) Synthesis of intermediate 6-26-2: the method is the same as the synthesis method of compound 6-4-4, except that compound 6-4-3 was replaced by compound 6-26-1 with a yield of 83%.
2) Synthesis of intermediate 6-26-3: the method is the same as the synthesis method of compound 6-4-2, except that compound 6-4-1 was replaced by compound 6-26-2 with a yield of 85%.
3) Synthesis of intermediate 6-26-4: the method is the same as the synthesis method of compound 6-4-4, except that compounds 6-4-2 and 6-4-3 were replaced by compounds 6-26-3 and 6-16-1, respectively, and the yield was 72%.
4) Synthesis of Compound 6-26: the method is the same as the synthesis method of compound 6-4, except that compounds 6-4-4 and 6-4-5 were replaced by compounds 6-26-4 and 6-16-3, respectively, and the yield was 73%. MS (ASAP): 671.

### Example 11

### Synthesis route of compound (6-37):

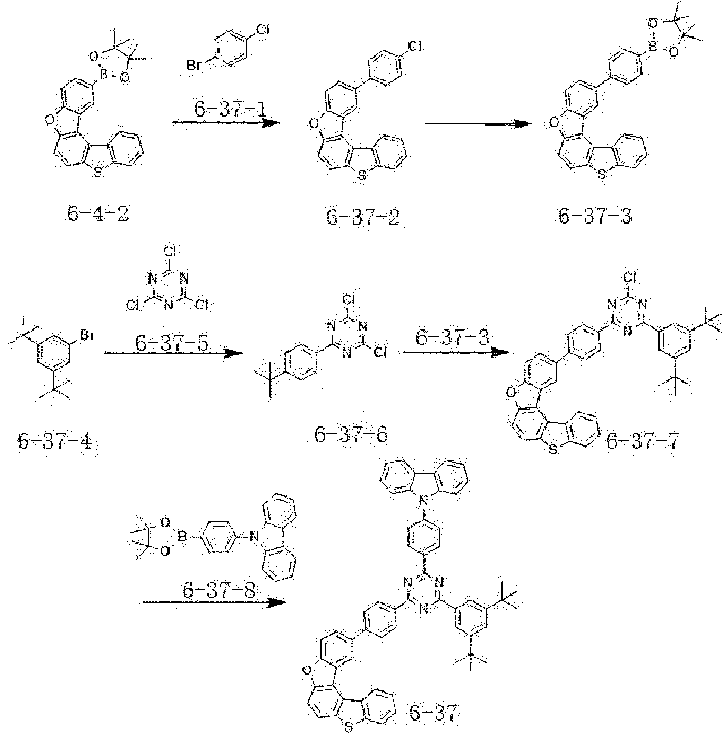

1) Synthesis of intermediate 6-37-2: the method is the same as the synthesis method of compound 6-4-4, except that compound 6-4-3 was replaced by compound 6-37-1 with a yield of 85%.
2) Synthesis of intermediate 6-37-3: the method is the same as the synthesis method of compound 6-4-2, except that compound 6-4-1 was replaced by compound 6-37-2 with a yield of 86%.
3) Synthesis of intermediate 6-37-6: compound 6-37-4 (13.5 g, 50 mmol) and anhydrous tetrahydrofuran (150 mL) were added to a three-necked flask of 300 mL under nitrogen atmosphere, stirred and dissolved, then cooled to a temperature of -78 °C, and 50 mL of n-butyllithium was slowly added dropwise, the reaction was carried out for 2 hours under stirring. After that, compound 6-37-5 (13.5 g, 50 mmol) was further added at one time, and the reaction was continued for 6 hours. After the reaction was completed, the reaction was quenched by adding aqueous sodium bisulfite solution to the reaction solution. The obtained reaction solution was extracted with dichloromethane and washed with water for three times to collect the organic solution. The organic solution was then purified on a silica gel column through column chromatography with a yield of 76%.
4) Synthesis of intermediate 6-37-7: the method is the same as the synthesis method of compound 6-4-4, except that compounds 6-4-2 and 6-4-3 were replaced by compounds 6-37-3 and 6-37-6, respectively, and the yield was 73%.
5) Synthesis of compound 6-37: the method is the same as the synthesis method of compound 6-4-4, except that compounds 6-4-2 and 6-4-3 were replaced by compounds 6-37-8 and 6-37-7, respectively, and the yield was 75%. MS (ASAP): 859.

### Example 12

### Synthesis route of compound (6-43):

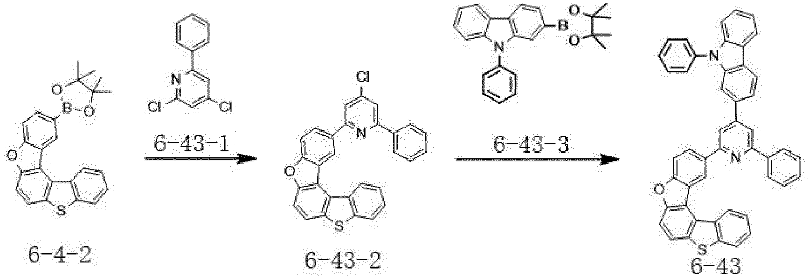

1) Synthesis of intermediate 6-43-2: the method is the same as the synthesis method of compound 6-4-4, except that compound 6-4-3 was replaced by compound 6-43-1 with a yield of 67%.
2) Synthesis of Compound 6-43: the method is the same as the synthesis method of compound 6-4-4, except that compounds 6-4-2 and 6-4-3 were replaced by compounds 6-43-3 and 6-43-2, respectively, and the yield was 75%. MS (ASAP): 669.

### Example 13

### Synthesis route of compound (6-50):

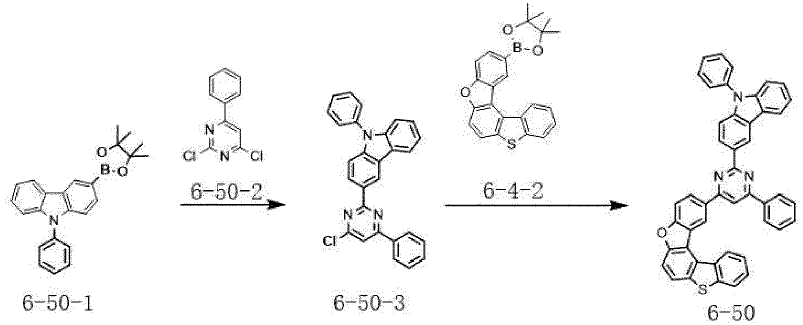

1) Synthesis of intermediate 6-50-3: the method is the same as the synthesis method of compound 6-4-4, except that compounds 6-4-2 and 6-4-3 were replaced by compounds 6-50-1 and 6-50-2, respectively, and the yield was 69%.
2) Synthesis of compound 6-50: the method is the same as the synthesis method of compound 6-4-4, except that compound 6-4-4 was replaced by compound 6-50-3 with a yield of 72%. MS (ASAP): 670.

### Example 14

### Synthesis route of compound (6-58):

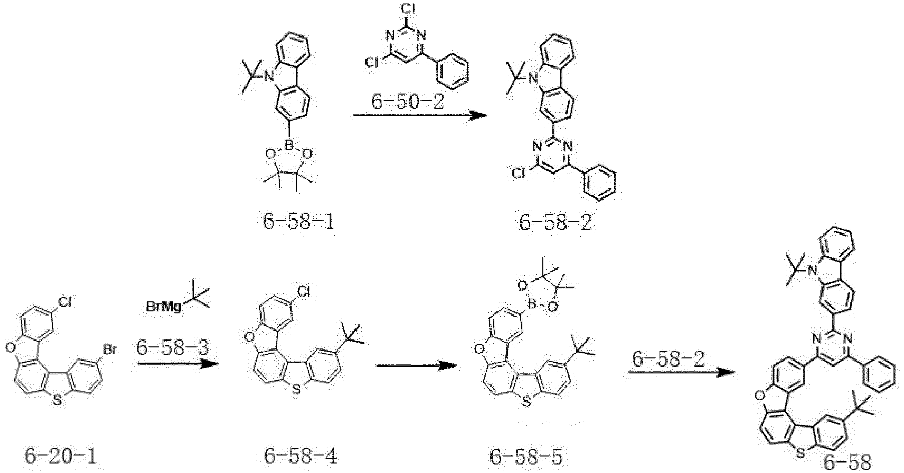

1) Synthesis of intermediate 6-58-2: the method is the same as the synthesis method of compound 6-4-4, except that compounds 6-4-2 and 6-4-3 were replaced by compounds 6-58-1 and 6-50-2, respectively, and the yield was 67%.
2) Synthesis of intermediate 6-58-4: compound 6-20-1 (19.4 g, 50 mmol) and anhydrous tetrahydrofuran (150 mL) were added to a three-necked flask of 300 mL under nitrogen atmosphere, stirred and dissolved, followed by adding 50 mL of compound 6-58-3 slowly dropwise in an ice bath, the reaction was carried out for 6 hours under stirring. After the reaction was completed, it was quenched by adding aqueous ammonium chloride solution to the reaction solution. The resulting reaction solution was extracted with dichloromethane and washed with water for three times to collect the organic solution. The organic solution was then purified on a silica gel column through column chromatography with a yield of 77%.
3) Synthesis of intermediate 6-58-5: the method is the same as the synthesis method of compound 6-4-2, except that compound 6-4-1 was replaced by compound 6-58-4 with a yield of 82%.
4) Synthesis of Compound 6-58: the method is the same as the synthesis method of compound 6-4-4, except that compounds 6-4-2 and 6-4-3 were replaced by compounds 6-58-5 and 6-58-2, respectively, and the yield was 70%. MS (ASAP): 706.

### Example 15

### Synthesis route of compound (6-60):

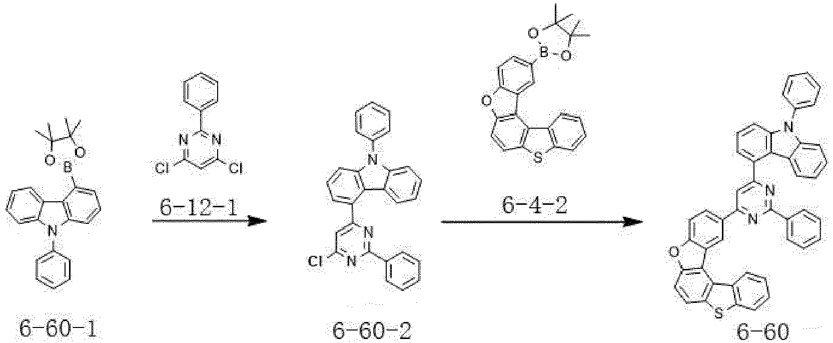

1) Synthesis of intermediate 6-60-2: the method is the same as the synthesis method of compound 6-4-4, except that compounds 6-4-2 and 6-4-3 were replaced by compounds 6-60-1 and 6-12-1, respectively, and the yield was 66%.
2) Synthesis of compound 6-60: the method is the same as the synthesis method of compound 6-4-4, except that compound 6-4-3 was replaced by compound 6-60-2 with a yield of 71%. MS (ASAP): 670.

### Example 16

### Synthesis route of compound (6-64):

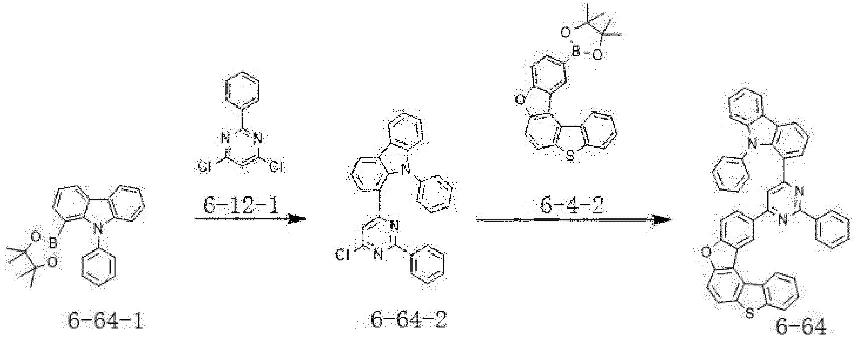

1) Synthesis of intermediate 6-64-2: the method is the same as the synthesis method of compound 6-4-4, except that compounds 6-4-2 and 6-4-3 were replaced by compounds 6-64-1 and 6-12-1, respectively, and the yield was 65%.
2) Synthesis of compound 6-64: the method is the same as the synthesis method of compound 6-4-4, except that compound 6-4-3 was replaced by compound 6-64-2 with a yield of 70%. MS (ASAP): 670.

### Example 17

### Synthesis route of compound (6-72):

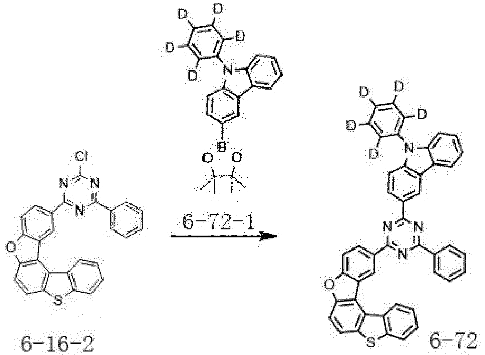

1) Synthesis of compound 6-72: the method is the same as the synthesis method of compound 6-4-4, except that compounds 6-4-2 and 6-4-3 were replaced by compounds 6-72-1 and 6-16-2, respectively, and the yield was 73%. MS (ASAP): 676.

### Example 18

### Synthesis route of compound (6-74):

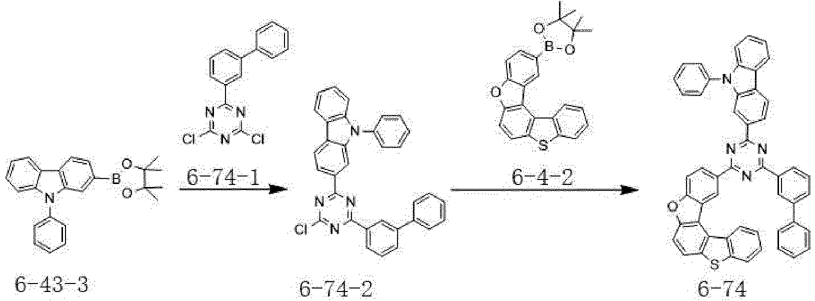

1) Synthesis of intermediate 6-74-2: the method is the same as the synthesis method of compound 6-4-4, except that compounds 6-4-2 and 6-4-3 were replaced by compounds 6-43-3 and 6-74-1, respectively, and the yield was 66%.
2) Synthesis of compound 6-74: the method is the same as the synthesis method of compound 6-4-4, except that compound 6-4-3 was replaced by compound 6-74-2 with a yield of 72%. MS (ASAP): 747.

### Example 19

### Synthesis route of compound (6-75):

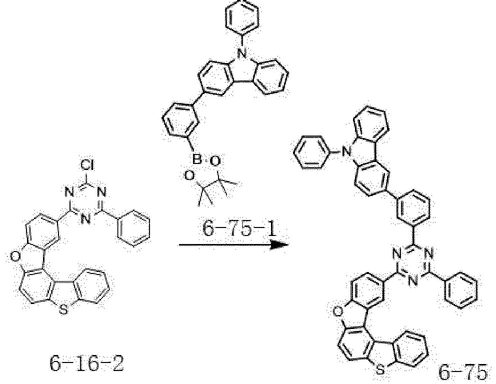

1) Synthesis of Compound 6-75: the method is the same as the synthesis method of compound 6-4-4, except that compounds 6-4-2 and 6-4-3 were replaced by compounds 6-75-1 and 6-16-2, respectively, and the yield was 74%. MS (ASAP): 747.

### Example 20

### Synthesis route of compound (6-77):

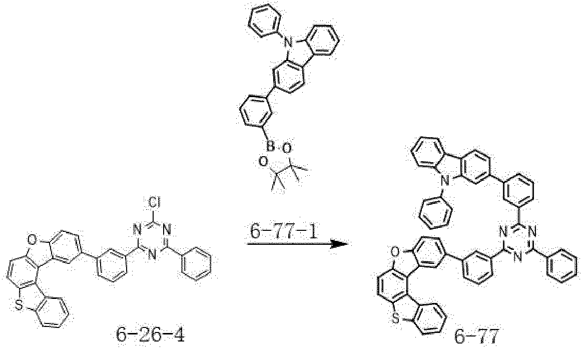

1) Synthesis of Compound 6-77: the method is the same as the synthesis method of compound 6-4-4, except that compounds 6-4-2 and 6-4-3 were replaced by compounds 6-77-1 and 6-26-4, respectively, and the yield was 72%. MS (ASAP): 823.

### Example 21

### Synthesis route of compound (6-79):

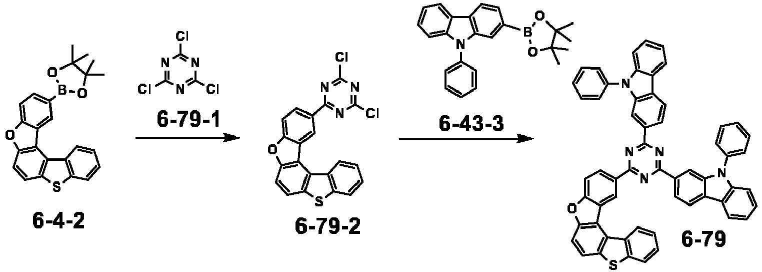

1) Synthesis of intermediate 6-79-2: the method is the same as the synthesis method of compound 6-4-4, except that compound 6-4-3 was replaced by compound 6-79-1 with a yield of 65%.
2) Synthesis of compound 6-79: the method is the same as the synthesis method of compound 6-4-4, except that compound 6-4-3 and 6-4-2 were replaced by compound 6-79-2 and double molar amount of compound 6-43-3, respectively, and the yield was 74%. MS (ASAP): 836.

### Example 22

### Synthetic route of compound (5-12):

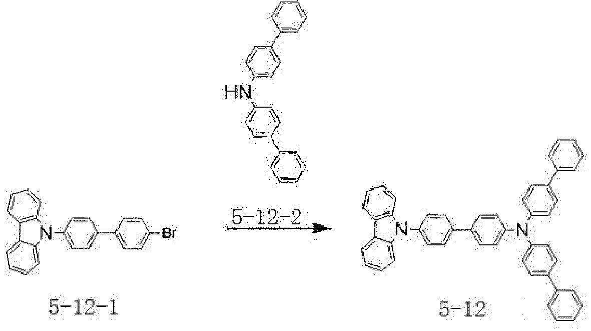

1) Synthesis of Compound 5-12: compound 5-12-1 (19.9 g, 50 mmol), compound 5-12-2 (16.1 g, 50 mmol), compound Pd₂(dba)₃ (0.55 g, 0.6 mmol), compound tri-tert-butylphosphine (0.24 g, 1.2 mmol), compound sodium tert-butoxide (4.1 g, 45 mmol), and 100 mL of anhydrous toluene solvent were added to a two-necked flask of 300 mL, which were heated under a temperature of 60 °C, and stirred for 6 hours, followed by cooling to room temperature, and quenching with water. After most of the solvent was removed through rotary evaporation, the resulting reaction solution was dissolved with dichloromethane, and washed with water for three times to collect the organic solution. The organic solution was then purified on a silica gel column through column chromatography with a yield of 75%. MS (ASAP): 639.

### Example 23

### Synthesis route of compound (5-45):

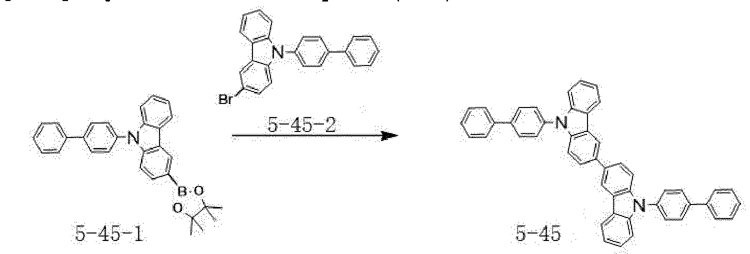

1) Synthesis of Compound 5-45: the method is the same as the synthesis method of compound 6-4-4, except that compounds 6-4-2 and 6-4-3 were replaced by compounds 5-45-1 and 5-45-2, respectively, and the yield was 81%. MS (ASAP): 637.

### Example 24

### Synthesis route of compound (5-46):

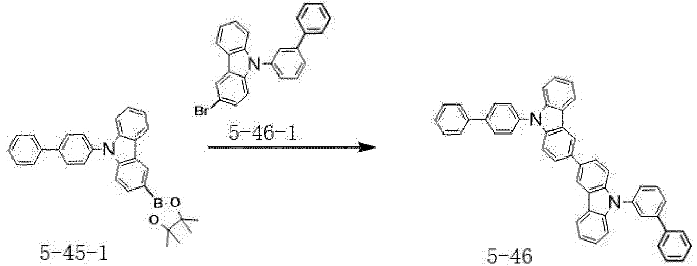

1) Synthesis of Compound 5-46: the method is the same as the synthesis method of compound 6-4-4, except that compounds 6-4-2 and 6-4-3 were replaced by compounds 5-45-1 and 5-46-1, respectively, and the yield was 80%. MS (ASAP): 637.

### Example 25

### Synthesis route of compound (5-47):

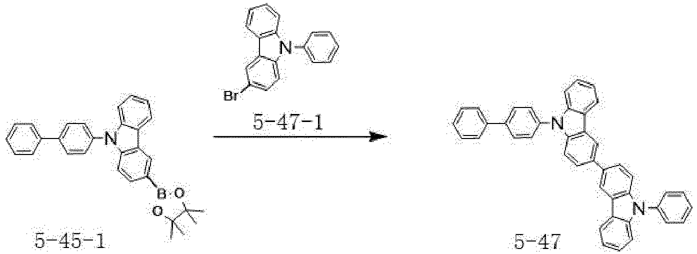

1) Synthesis of Compound 5-47: the method is the same as the synthesis method of compound 6-4-4, except that compounds 6-4-2 and 6-4-3 were replaced by compounds 5-45-1 and 5-47-1, respectively, and the yield was 83%. MS (ASAP): 561.

### Example 26

### Synthesis route of compound (5-50):

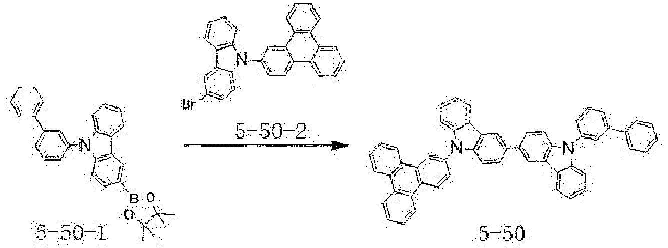

1) Synthesis of Compound 5-50: the method is the same as the synthesis method of compound 6-4-4, except that compounds 6-4-2 and 6-4-3 were replaced by compounds 5-50-1 and 5-50-2, respectively, and the yield was 83%. MS (ASAP): 711.

### Example 27

### Synthesis route of compound (5-88):

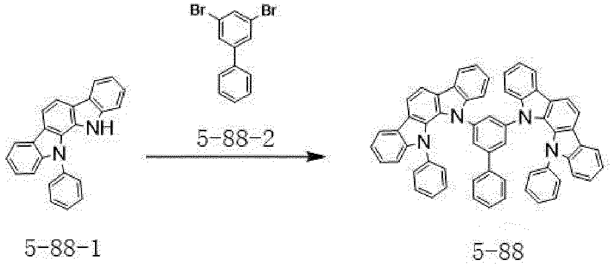

1) Synthesis of Compound 5-88: the method is the same as the synthesis method of compound 5-12, except that compounds 5-12-1 and 5-12-2 were replaced by compounds 5-88-2 and double amounts of 5-88-1, and the yield was 75%. MS (ASAP): 815.

### Example 28

### Synthesis route of compound (5-89):

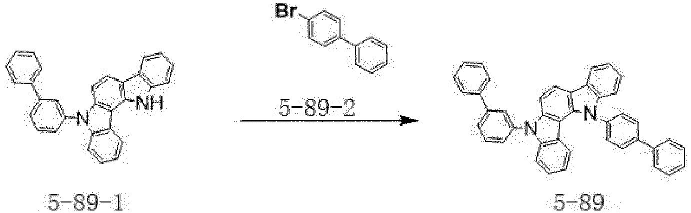

1) Synthesis of compound 5-89: the method is the same as the synthesis method of compound 5-12, except that compounds 5-12-1 and 5-12-2 were replaced by compounds 5-89-2 and 5-89-1, and the yield was 80%. MS (ASAP): 561.

### Comparative compounds are as follows:

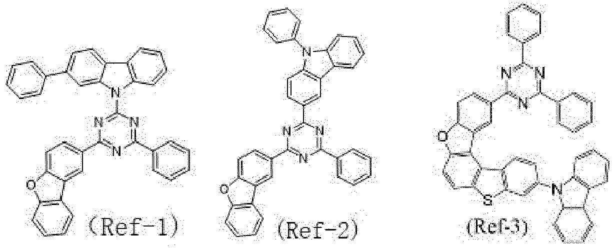

### Preparation and characterization of OLED devices:

In this example, in a green light device, the host material is selected from the heterocycle-containing organic compound in the present application or a mixture thereof, HATCN is used as the hole injection layer material, HT is used as the hole transport material, GP is used as the electron blocking layer material, GD is used as the doping material of the light-emitting material, ET is used as the electron transport material, and Liq (8-hydroxyquinoline lithium) is used as the electron injection material. The device has a structure of ITO/HATCN/HT/GP/host material: GD (8%wt)/ET:Liq/Liq/Al.

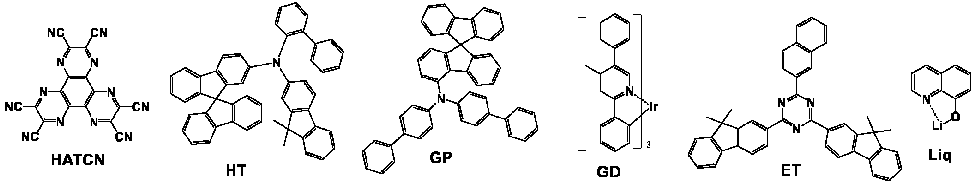

The above-mentioned materials such as HATCN, HT, GP, GD, ET and Liq are all commercially available or their synthetic methods existing technologies.

The preparation process of the OLED devices using the above-mentioned materials will be described in detail by means of specific examples.

### Device Example 1

The method of manufacturing the OLED devices in this example includes the steps as follows:
1) Cleaning of the ITO (indium tin oxide) anode layer: cleaning the ITO conductive glass anode layer, followed by ultrasonic cleaning with deionized water, acetone, and isopropanol for 15 minutes, and then treating in a plasma cleaner for 5 minutes to improve the work function of the electrode.
2) Forming a hole injection layer by subjecting a hole injection layer material HATCN to evaporation through vacuum evaporation on the ITO anode layer at an evaporation rate of 1 Å/s, which has a thickness of 30 nm.
3) Forming a hole transport layer by subjecting a hole transport material HT to evaporation through vacuum evaporation on the hole injection layer, which has a thickness of 60 nm.
4) Forming an electron blocking layer by subjecting an electron blocking layer material GP to evaporation on the hole transport layer, which has a thickness of 10 nm.
5) Forming a light-emitting layer by subjecting a light-emitting layer to evaporation on an electron blocking layer, wherein the compound (6-4) is used as a host material GH, GD is used as a doping material, and the mass ratio of GD and GH is 8: 100, it has a thickness of 40 nm.
6) Forming an electron transport layer by subjecting the electron transport materials ET and LiQ (at a ratio of 5: 5 (mass ratio)) to evaporation through vacuum evaporation on the light-emitting layer, which has a thickness of 30 nm.
7) Forming an electron injection layer by subjecting an electron injection layer LiQ to vacuum evaporation on the electron transport layer, which has a thickness of 1 nm.
8) Forming a cathode layer by subjecting a cathode Al layer to vacuum evaporation on the electron injection layer, which has a thickness of 100 nm.

### Device Example 2-21

The OLED device is manufactured by using the corresponding host material as described in Table 1 as the host material GH of the OLED devices instead of (6-4) in Device Example 1, and other conditions remain unchanged.

### Device Comparative Example 1

The OLED device is manufactured by using compound (Ref-1) as the host material GH of the OLED device instead of (6-4) in Device Example 1, and other conditions remain unchanged.

### Device Comparative Example 2

The OLED device is manufactured by using compound (Ref-2) as the host material GH of the OLED device instead of (6-4) in Device Example 1, and other conditions remain unchanged.

Device Comparative Example 3

The OLED device is manufactured by using compound (Ref-3) as the host material GH of the OLED device instead of (6-4) in Device Example 1, and other conditions remain unchanged.

The current voltage (J-V) characteristics of the organic light-emitting diodes of green device in Examples 1 to 21 and Comparative Examples 1 to 3 are tested using a characterization device, while important parameters such as efficiency, lifetime (see Table 1) and external quantum efficiency are recorded as well. In Table 1, the luminous efficiency is the relative value obtained when the current density is 10 mA/cm², and all the luminous efficiencies and the lifetime are relative to the organic light-emitting diode of Comparative Example 2. It can be seen that the efficiencies and lifetime of the embodiments in the present application are significantly improved compared to the comparative examples. In examples of the present application, the heterocycle-containing organic compounds having the structure of formula (1) or (2) are used as the host materials of the light-emitting layer, while in comparative example, other organic compounds are used as the host materials of the light-emitting layer. It can be seen that the OLED devices prepared by using the compounds of the present application have greatly improved efficiencies and lifetime.

**Table 1**

| Serial number | Host materials | Luminous efficiency | Lifetime |
|---|---|---|---|
| Device Example 1 | (6-4) | 1.53 | 1.58 |
| Device Example 2 | (6-9) | 1.60 | 1.66 |
| Device Example 3 | (6-10) | 1.65 | 1.70 |
| Device Example 4 | (6-12) | 1.63 | 1.68 |
| Device Example 5 | (6-16) | 1.82 | 1.90 |
| Device Example 6 | (6-17) | 1.79 | 1.86 |
| Device Example 7 | (6-20) | 1.84 | 1.93 |
| Device Example 8 | (6-22) | 1.75 | 1.81 |
| Device Example 9 | (6-24) | 1.72 | 1.78 |
| Device Example 10 | (6-26) | 1.77 | 1.84 |
| Device Example 11 | (6-37) | 1.74 | 1.80 |
| Device Example 12 | (6-43) | 1.55 | 1.60 |
| Device Example 13 | (6-50) | 1.59 | 1.64 |
| Device Example 14 | (6-58) | 1.83 | 1.91 |
| Device Example 15 | (6-60) | 1.58 | 1.63 |
| Device Example 16 | (6-64) | 1.56 | 1.61 |
| Device Example 17 | (6-72) | 1.71 | 1.77 |
| Device Example 18 | (6-74) | 1.69 | 1.74 |
| Device Example 19 | (6-75) | 1.7 | 1.75 |
| Device Example 20 | (6-77) | 1.67 | 1.73 |
| Device Example 21 | (6-79) | 1.66 | 1.71 |
| Device Comparative Example 1 | (Ref-1) | 1.02 | 1.03 |
| Device Comparative Example 2 | (Ref-2) | 1 | 1 |
| Device Comparative Example 3 | (Ref-3) | 1.04 | 1.06 |

### Device Examples 22-42

The OLED device is manufactured by using the corresponding host materials as described in Table 2 as the host materials GH of the OLED devices instead of (6-4) in Device Example 1, and other conditions remain unchanged.

### Device Comparative Example 4

The OLED device is manufactured by using compounds (Ref-1): (5-45) (with a molar ratio of 4:6) as the host material of the OLED device instead of (6-4) in Device Example 1, and other conditions remain unchanged.

### Device Comparative Example 5

The OLED device is manufactured by using compounds (Ref-2): (5-45) (with a molar ratio of 4:6) as the host material of the OLED device instead of (6-4) in Device Example 1, and other conditions remain unchanged.

### Device Comparative Example 6

The OLED device is manufactured by using compounds (Ref-3): (5-88) (with a molar ratio of molar ratio 5:5) as the host material of the OLED device instead of (6-4) in Device Example 1, and other conditions remain unchanged.

The current voltage (J-V) characteristics of the organic light-emitting diodes of green device in Examples 22 to 42 and Comparative Examples 4 to 6 are tested using a characterization device, while important parameters such as efficiency, lifetime (see Table 2) and external quantum efficiency are recorded as well. In Table 2, the luminous efficiency is the relative value obtained when the current density is 10 mA/cm², and all the luminous efficiencies and the lifetime are relative to the organic light-emitting diode of Comparative Example 5. It can be seen that the efficiencies and lifetime of the embodiments in the present application are significantly improved compared to the comparative examples. In examples of the present application, the heterocycle-containing organic compounds having the structure of formula (1) or (2) are used as the host materials of the light-emitting layer, while in comparative example, other organic compounds are used as the host materials of the light-emitting layer. It can be seen that the OLED devices prepared by using the compounds of the present application have greatly improved efficiencies and lifetime.

**Table 2**

| Serial number | Host materials | Molar ratio | Luminous efficiency | Lifetime |
|---|---|---|---|---|
| Device Example 22 | (6-4): (5-45) | 4:6 | 1.67 | 1.74 |
| Device Example 23 | (6-9): (5-45) | 4:6 | 1.76 | 1.84 |
| Device Example 24 | (6-10): (5-47) | 4:6 | 1.79 | 1.87 |
| Device Example 25 | (6-12): (5-46) | 4:6 | 1.78 | 1.85 |
| Device Example 26 | (6-16): (5-46) | 4:6 | 1.95 | 2.08 |
| Device Example 27 | (6-17): (5-45) | 4:6 | 1.91 | 2.04 |
| Device Example 28 | (6-20): (5-46) | 4:6 | 1.97 | 2.12 |
| Device Example 29 | (6-22): (5-12) | 4:6 | 1.88 | 2.00 |
| Device Example 30 | (6-24): (5-45) | 4:6 | 1.86 | 1.96 |
| Device Example 31 | (6-26): (5-45) | 4:6 | 1.90 | 2.03 |
| Device Example 32 | (6-37): (5-88) | 4:6 | 1.87 | 1.98 |
| Device Example 33 | (6-43): (5-89) | 5:5 | 1.65 | 1.73 |
| Device Example 34 | (6-50): (5-45) | 4:6 | 1.75 | 1.80 |
| Device Example 35 | (6-58): (5-50) | 4:6 | 1.96 | 2.09 |
| Device Example 36 | (6-60): (5-50) | 5:5 | 1.72 | 1.77 |
| Device Example 37 | (6-64): (5-50) | 5:5 | 1.69 | 1.76 |
| Device Example 38 | (6-72): (5-50) | 4:6 | 1.85 | 1.95 |
| Device Example 39 | (6-74): (5-50) | 5:5 | 1.82 | 1.92 |
| Device Example 40 | (6-75): (5-50) | 4:6 | 1.84 | 1.93 |
| Device Example 41 | (6-77): (5-88) | 5:5 | 1.81 | 1.90 |
| Device Example 42 | (6-79): (5-88) | 5:5 | 1.80 | 1.92 |
| Device Comparative Example 4 | (Ref-1): (5-45) | 4:6 | 1.06 | 1.10 |
| Device Comparative Example 5 | (Ref-2): (5-45) | 4:6 | 1 | 1 |
| Device Comparative Example 6 | (Ref-3): (5-88) | 5:5 | 1.09 | 1.14 |

The heterocyclic group-containing organic compounds have good performance of transferring and adjusting charge balance, and can be used as host materials in an organic electronic device, thereby improving the luminous efficiency and the lifetime of the device.

In view of the foregoing, the heterocycle-containing organic compounds, mixtures, compositions, and organic electronic devices provided in examples of the present application have been described in detail above, and the principles and embodiments of the present application are described by using specific examples herein. Descriptions of the above examples are merely intended to help understand the technical solutions and core ideas of the present application. Meanwhile, for a person with ordinary skill in the art, various modifications may still be made to the technical solutions described in the foregoing examples, or equivalents may be made to the detailed embodiments and the scope of application in accordance with ideas of the present application. In view of the foregoing, the contents of the specification should not be construed as limiting the present application.

## Claims

1. A heterocyclic group-containing organic compound, wherein the heterocyclic group-containing organic compound has a structure represented by formula (1) or (2): 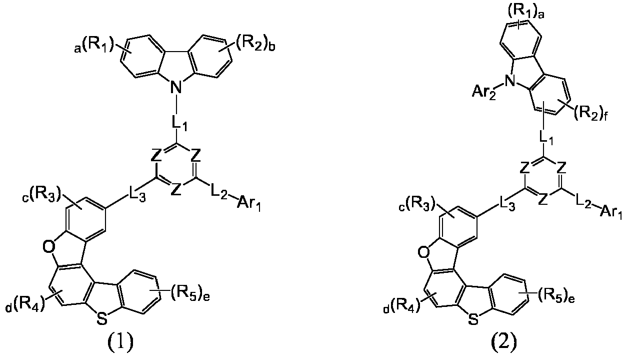 wherein:
Z is independently selected from CR₆ or an N atom, wherein at least one Z is an N atom;
R₁, R₂ or R₆ are each independently selected from -H, -D, or a linear alkyl group having 1 to 20 carbon atoms, a linear alkoxy group having 1 to 20 carbon atoms, or a linear thioalkoxy group having 1 to 20 carbon atoms, or a branched alkyl group having 3 to 20 carbon atoms, or a cyclic alkyl group having 3 to 20 carbon atoms, or a branched alkoxy group having 3 to 20 carbon atoms, or a cyclic alkoxy group having 3 to 20 carbon atoms, or a branched thioalkoxy group having 3 to 20 carbon atoms, or a cyclic thioalkoxy group having 3 to 20 carbon atoms, or a silyl group, or a ketone group having 1 to 20 carbon atoms, or an alkoxycarbonyl group having 2 to 20 carbon atoms, or an aryloxycarbonyl group having 7 to 20 carbon atoms, a cyano group, a carbamoyl group, a haloformyl group, a formyl group, an isocyano group, an isocyanate group, a thiocyanate group or an isothiocyanate group, a hydroxyl group, a nitro group, -CF₃, -Cl, -Br, -F, -I, or substituted or unsubstituted aromatic group having 6 to 60 ring atoms, or a substituted or unsubstituted heteroaromatic groups having 5 to 60 ring atoms, or a substituted or unsubstituted aryloxy group or heteroaryloxy group having 5 to 60 ring atoms, or a combinations thereof;
R₃, R₄ or R₅ are each independently selected from -H, -D, or a linear alkyl group having 1 to 20 carbon atoms, or a branched or cyclic alkyl group having 3 to 20 carbon atoms, or a phenyl group;
Ar₁ is selected from a substituted or unsubstituted aromatic group having 6 to 30 ring atoms, or a substituted or unsubstituted heteroaromatic group having 5 to 30 ring atoms;
Ar₂ is selected from -H, -D, or a linear alkyl group having 1 to 20 carbon atoms, or a branched or cyclic alkyl group having 3 to 20 carbon atoms, or a substituted or unsubstituted aromatic group having 6 to 30 ring atoms, or a substituted or unsubstituted heteroaromatic group having 5 to 30 ring atoms;
L₁, L₂ and L₃ are each independently selected from a single bond, or a substituted or unsubstituted aromatic group having 6 to 30 ring atoms, or a substituted or unsubstituted heteroaromatic group having 5 to 30 ring atoms; and
a, b and e are each independently selected from 0, 1, 2, 3, or 4; d is selected from 0, 1 or 2; and c and f are each independently selected from 0, 1, 2 or 3.

2. The heterocyclic group-containing organic compound according to claim 1, wherein the structure of the heterocyclic group-containing organic compound is selected from one of formulas (3-1) to (3-14):

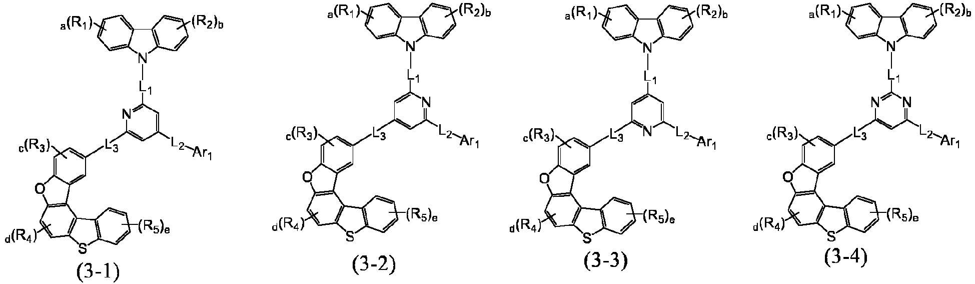

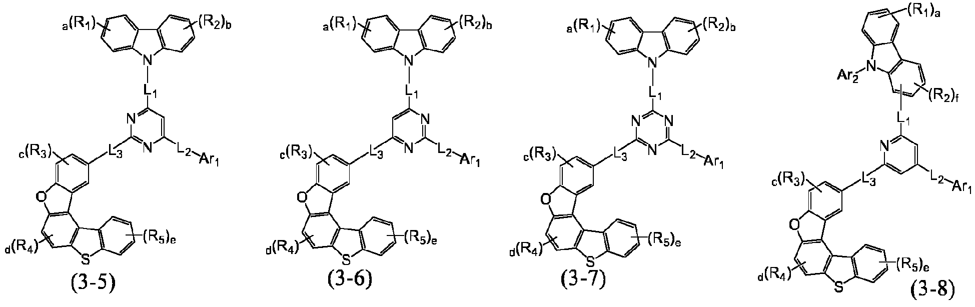

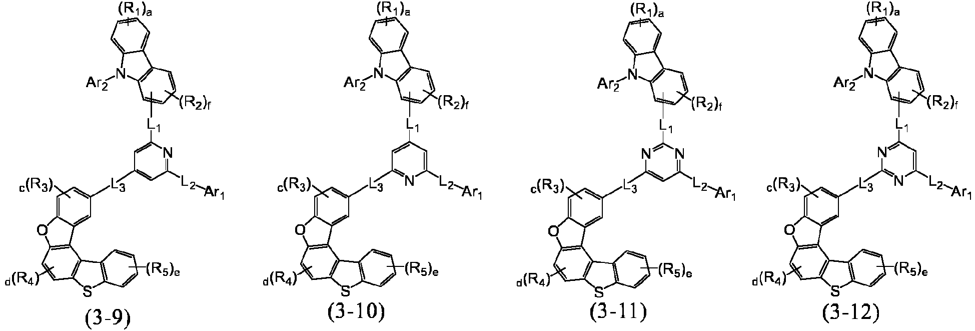

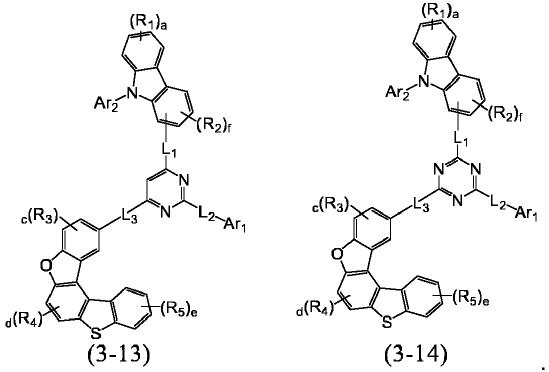

3. The heterocyclic group-containing organic compound according to claim 1, wherein the L₁, L₂ and L₃ are each independently selected from a single bond, a substituted or unsubstituted phenyl, a substituted or unsubstituted biphenyl, a substituted or unsubstituted terphenyl, a substituted or unsubstituted pyridyl, a substituted or unsubstituted pyrimidinyl, a substituted or unsubstituted triazinyl, and a substituted or unsubstituted naphthyl; and a substituted substituent is selected from -D, or a linear alkyl group having 1 to 10 carbon atoms, or a branched or cyclic alkyl group having 3 to 10 carbon atoms, or a phenyl group, or a naphthyl group.

4. The heterocyclic group-containing organic compound according to claim 3, wherein the structure of the heterocyclic group-containing organic compound is selected from one of formulas (4-1) to (4-8):

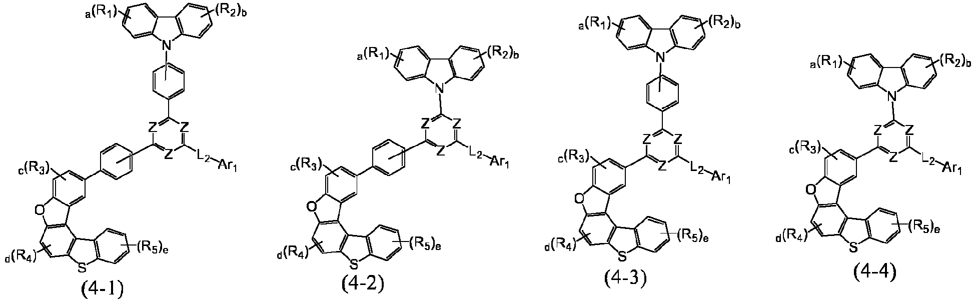

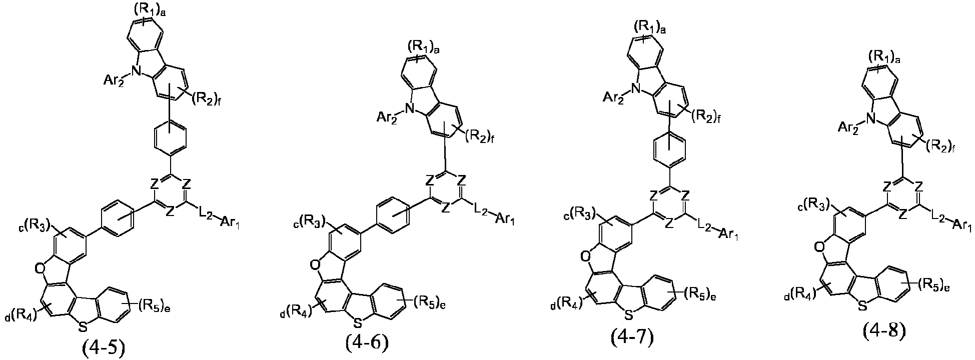

5. The heterocyclic group-containing organic compound according to claim 1, wherein Ar₁ is selected from one of following structures or a combination thereof: 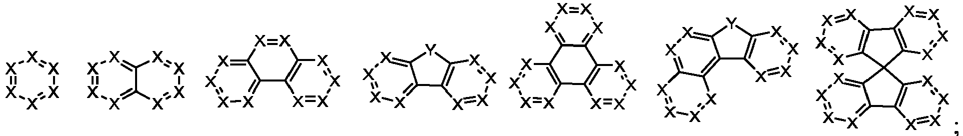 wherein:
X is independently selected from CR₇ or N;
Y is independently selected from C(R₈R₉), NR₈, Si(R₈R₉), O, S, Se, S=O, S(=O)₂, or PR₈;
R₇ to R₉ is independently selected from -H, -D, or a linear alkyl group having 1 to 20 carbon atoms, or a linear alkoxy group having 1 to 20 carbon atoms, or a linear thioalkoxy group having 1 to 20 carbon atoms, or a branched alkyl group having 3 to 20 carbon atoms, or a cyclic alkyl group having 3 to 20 carbon atoms, or a branched alkoxy group having 3 to 20 carbon atoms, or a cyclic alkoxy group having 3 to 20 carbon atoms, or a branched thioalkoxy group having 3 to 20 carbon atoms, or a cyclic thioalkoxy group having 3 to 20 carbon atoms, or a silyl group, or a ketone group having 1 to 20 carbon atoms, or an alkoxycarbonyl group having 2 to 20 carbon atoms, or an aryloxycarbonyl group having 7 to 20 carbon atoms, a cyano group, a carbamoyl group, a haloformyl group, a formyl group, an isocyano group, an isocyanate group, a thiocyanate group or an isothiocyanate group, a hydroxyl group, a nitro group, -CF₃, -Cl, -Br, -F, -I, or an aromatic group having 6 to 60 ring atoms, or a substituted aromatic group having 6 to 60 ring atoms, or a heteroaromatic group having 5 to 60 ring atoms, or a substituted heteroaromatic group having 5 to 60 ring atoms, or an aryloxy group having 6 to 60 ring atoms, or a heteroaryloxy group having 5 to 60 ring atoms, or a combinations thereof; and/or, adjacent R₇ form a ring with each other.

6. The heterocyclic group-containing organic compound according to claim 1, wherein Ar₂ is selected from methyl, ethyl, n-propyl, isopropyl, n-butyl, sec-butyl, tert-butyl, isobutyl, phenyl, or naphthyl, or a combination thereof.

7. The heterocyclic group-containing organic compound according to claim 1, wherein the heterocyclic group-containing organic compound is selected from any one of the following compounds:

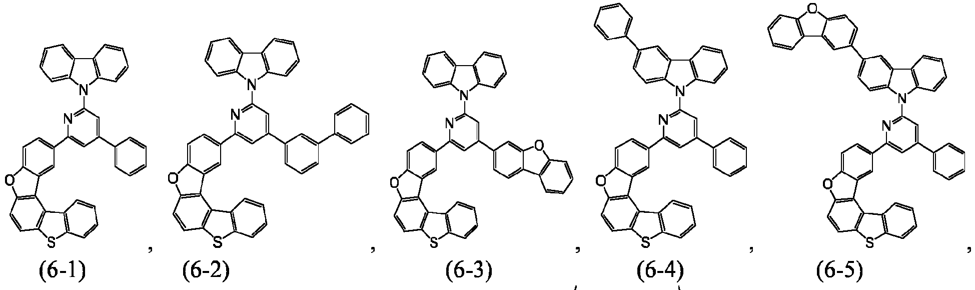

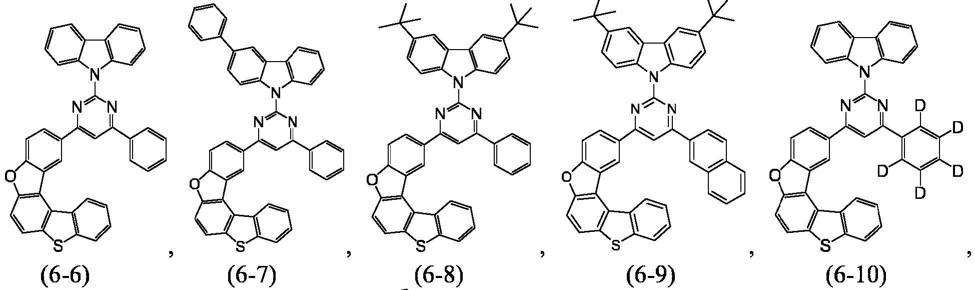

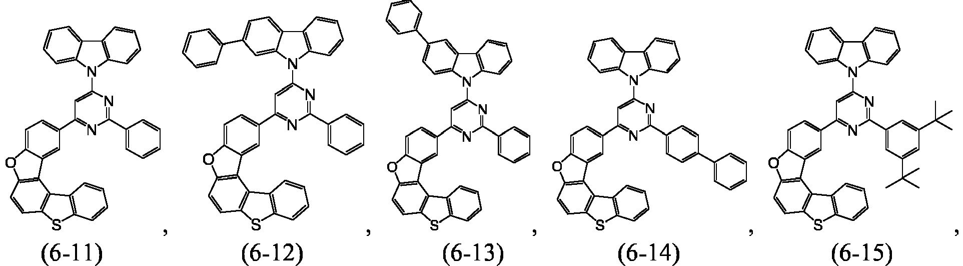

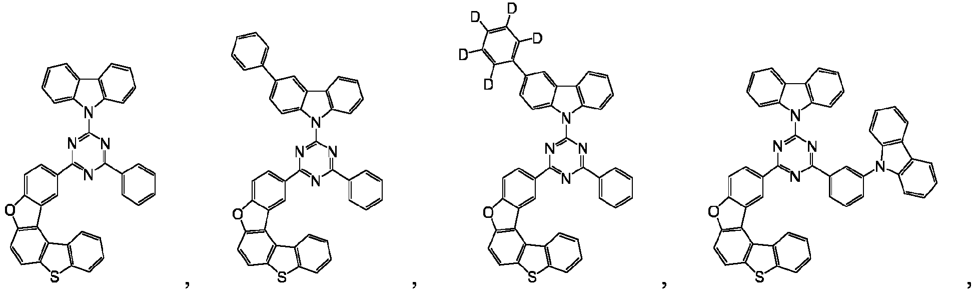

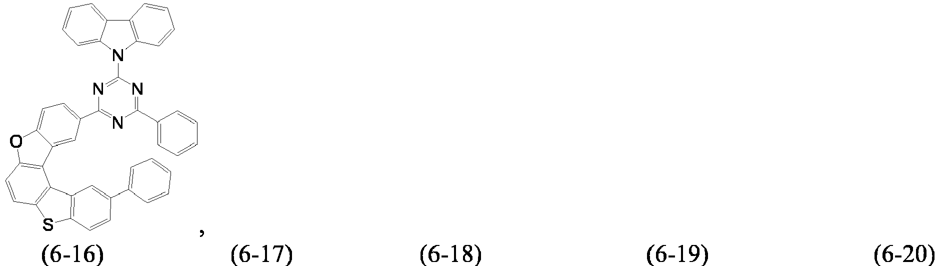

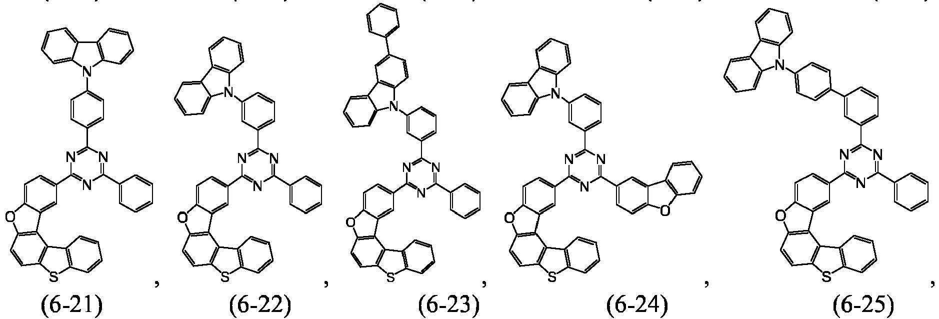

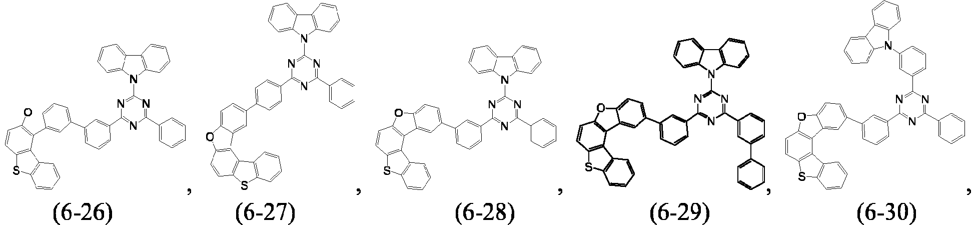

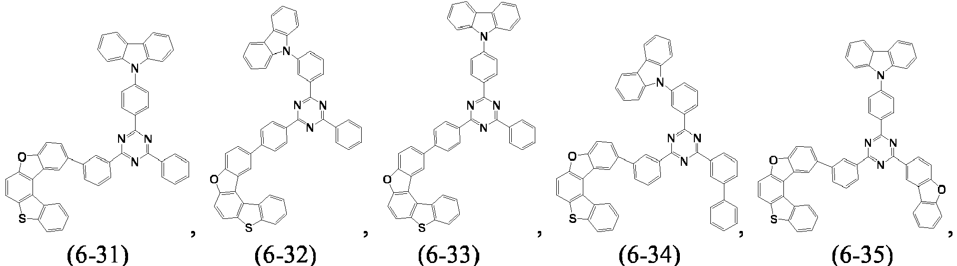

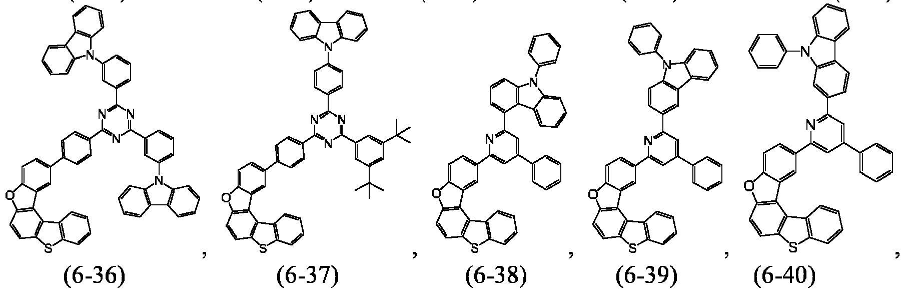

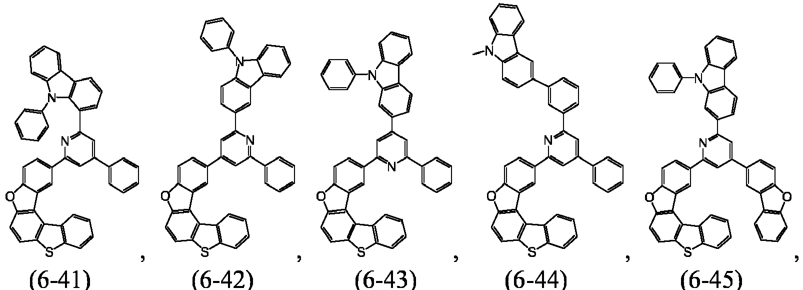

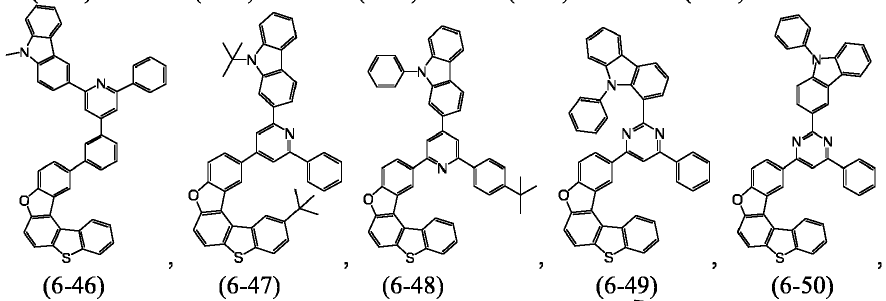

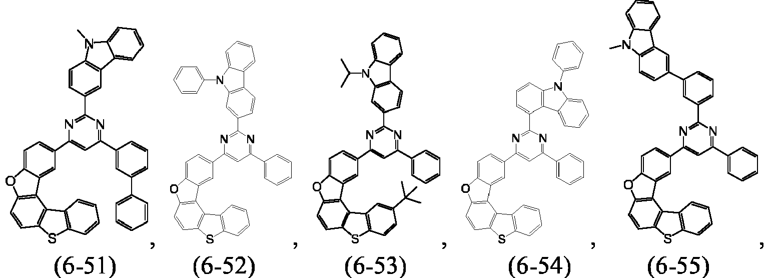

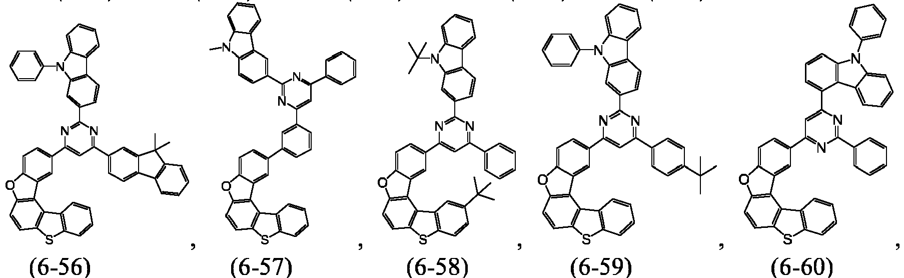

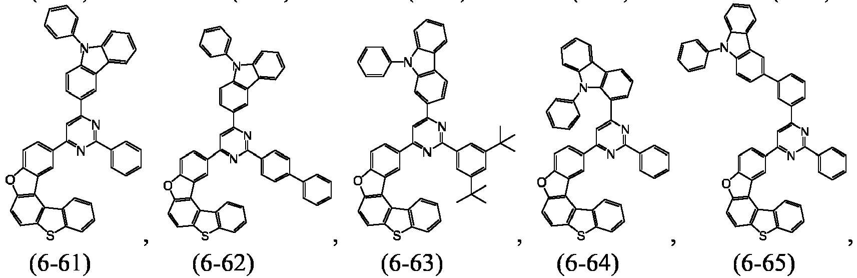

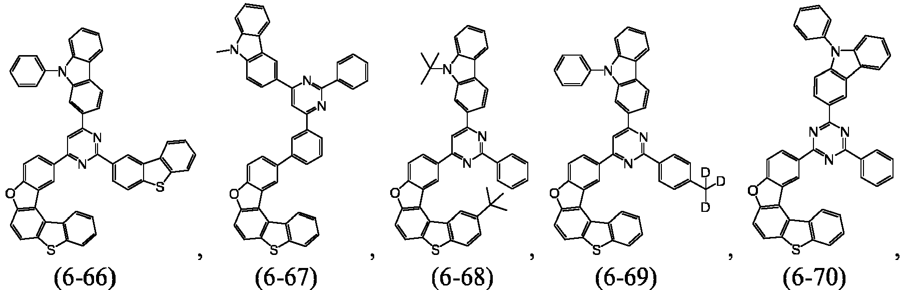

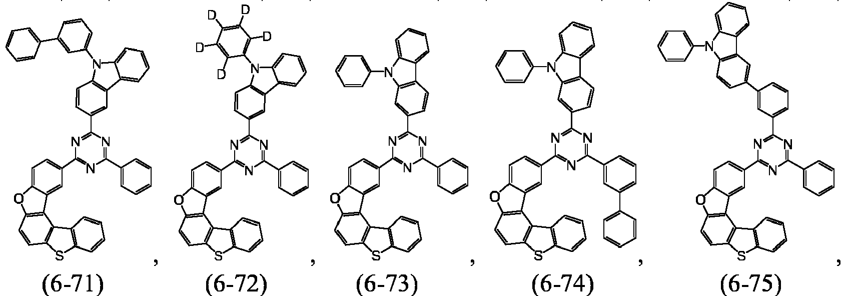

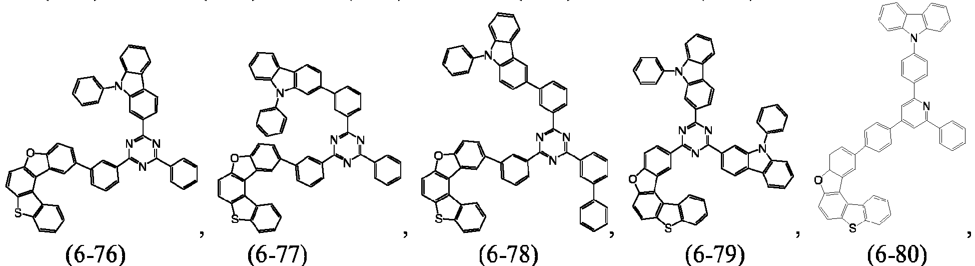

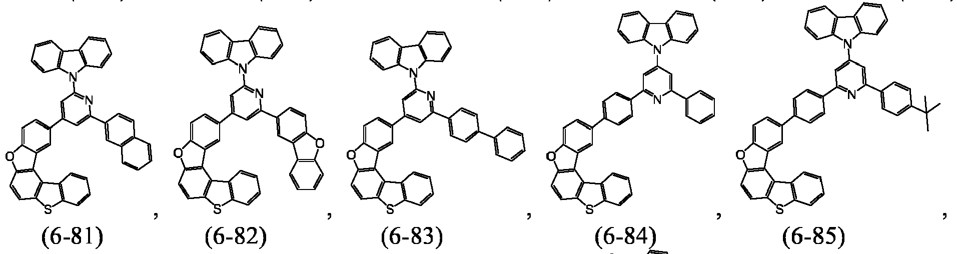

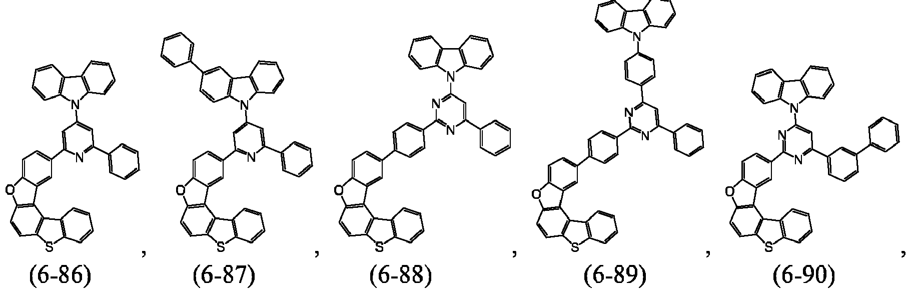

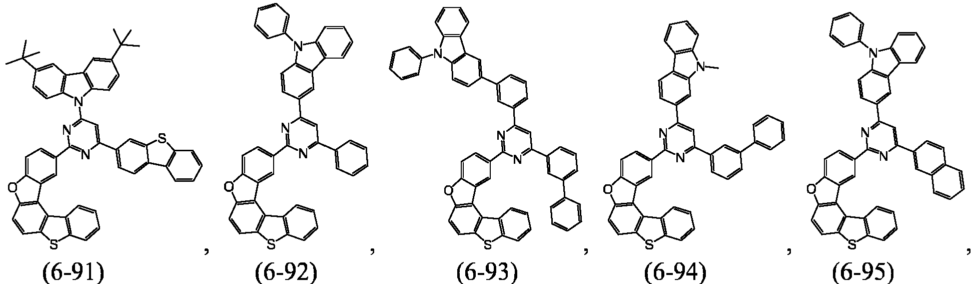

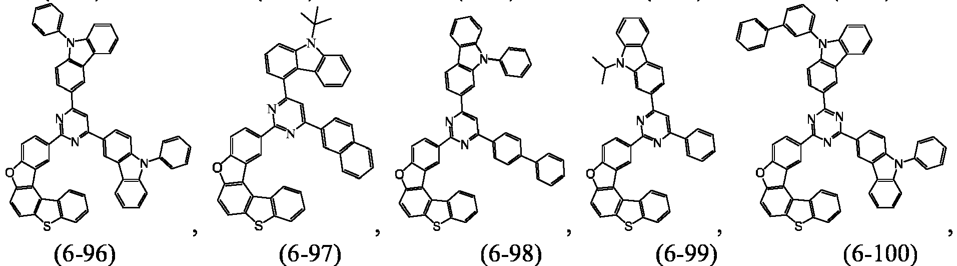

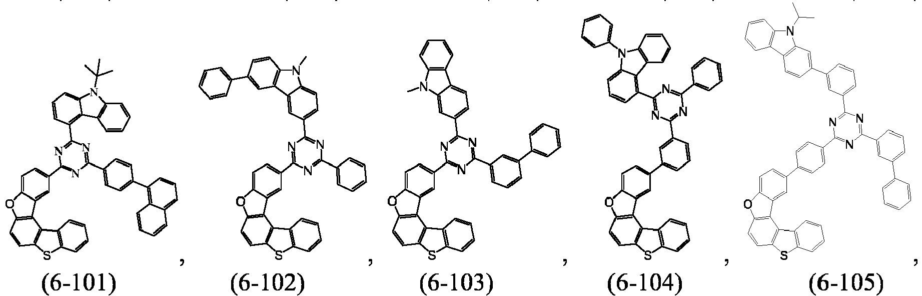

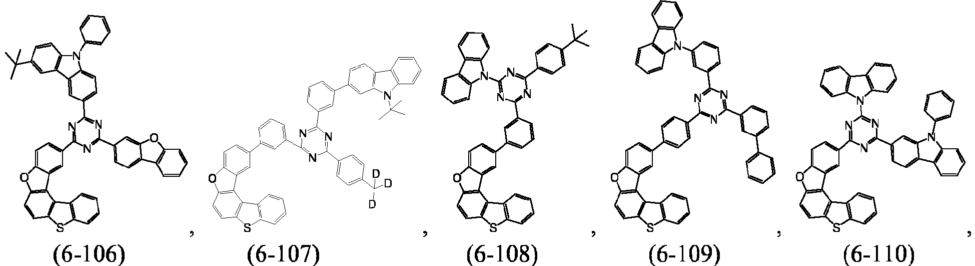

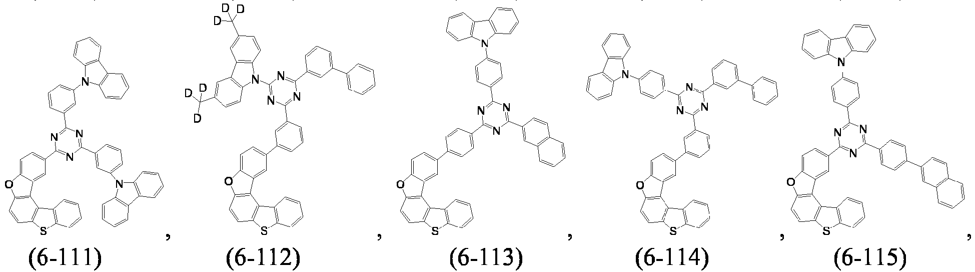

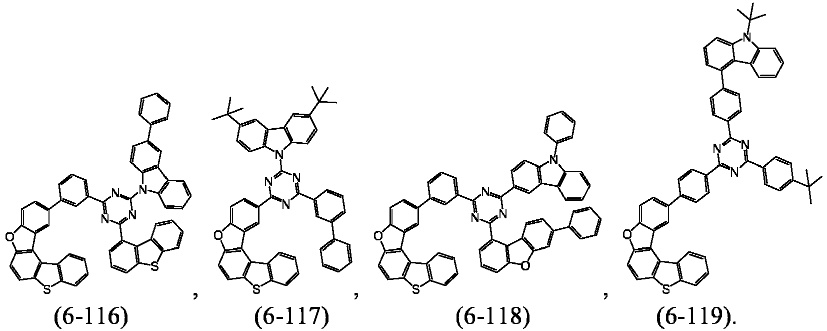

8. A mixture, wherein the mixture comprises at least one heterocyclic group-containing organic compound and at least one organic functional material, the at least one organic functional material is selected from a hole injection material, a hole transport material, an electron transport material, an electron injection material, an electron blocking material, a hole blocking material, a light-emitting material, a host material, or an organic dye; and the heterocyclic group-containing organic compound has a structure represented by formula (1) or (2): 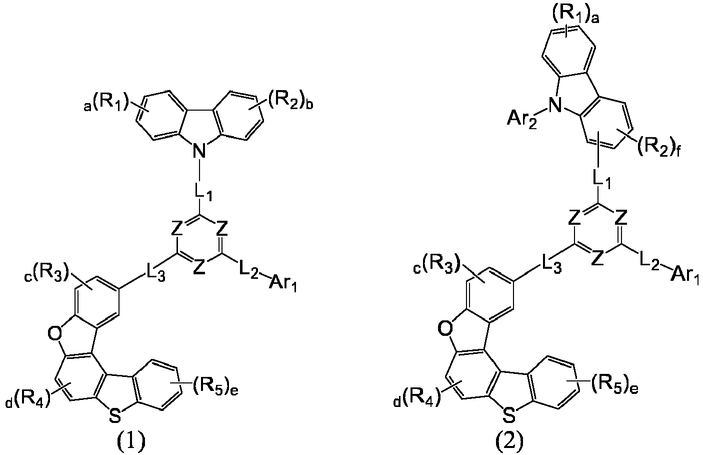 wherein:
Z is independently selected from CR₆ or an N atom, wherein at least one Z is an N atom;
R₁, R₂ or R₆ are each independently selected from -H, -D, or a linear alkyl group having 1 to 20 carbon atoms, a linear alkoxy group having 1 to 20 carbon atoms, or a linear thioalkoxy group having 1 to 20 carbon atoms, or a branched alkyl group having 3 to 20 carbon atoms, or a cyclic alkyl group having 3 to 20 carbon atoms, or a branched alkoxy group having 3 to 20 carbon atoms, or a cyclic alkoxy group having 3 to 20 carbon atoms, or a branched thioalkoxy group having 3 to 20 carbon atoms, or a cyclic thioalkoxy group having 3 to 20 carbon atoms, or a silyl group, or a ketone group having 1 to 20 carbon atoms, or an alkoxycarbonyl group having 2 to 20 carbon atoms, or an aryloxycarbonyl group having 7 to 20 carbon atoms, a cyano group, a carbamoyl group, a haloformyl group, a formyl group, an isocyano group, an isocyanate group, a thiocyanate group or an isothiocyanate group, a hydroxyl group, a nitro group, -CF₃, -Cl, -Br, -F, -I, or substituted or unsubstituted aromatic group having 6 to 60 ring atoms, or a substituted or unsubstituted heteroaromatic groups having 5 to 60 ring atoms, or a substituted or unsubstituted aryloxy group or heteroaryloxy group having 5 to 60 ring atoms, or a combinations thereof;
R₃, R₄ or R₅ are each independently selected from -H, -D, or a linear alkyl group having 1 to 20 carbon atoms, or a branched or cyclic alkyl group having 3 to 20 carbon atoms, or a phenyl group;
An is selected from a substituted or unsubstituted aromatic group having 6 to 30 ring atoms, or a substituted or unsubstituted heteroaromatic group having 5 to 30 ring atoms;
Ar₂ is selected from -H, -D, or a linear alkyl group having 1 to 20 carbon atoms, or a branched or cyclic alkyl group having 3 to 20 carbon atoms, or a substituted or unsubstituted aromatic group having 6 to 30 ring atoms, or a substituted or unsubstituted heteroaromatic group having 5 to 30 ring atoms;
L₁, L₂ and L₃ are each independently selected from a single bond, or a substituted or unsubstituted aromatic group having 6 to 30 ring atoms, or a substituted or unsubstituted heteroaromatic group having 5 to 30 ring atoms; and
a, b and e are each independently selected from 0, 1, 2, 3, or 4; d is selected from 0, 1 or 2; and c and f are each independently selected from 0, 1, 2 or 3.

9. The mixture according to claim 8, wherein the structure of the heterocyclic group-containing organic compound is selected from one of formulas (3-1) to (3-14):

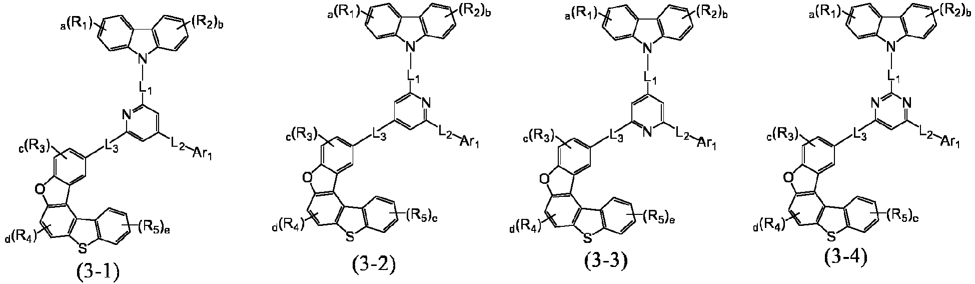

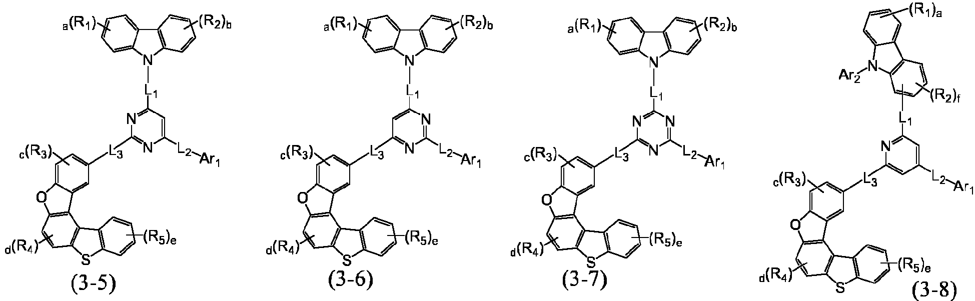

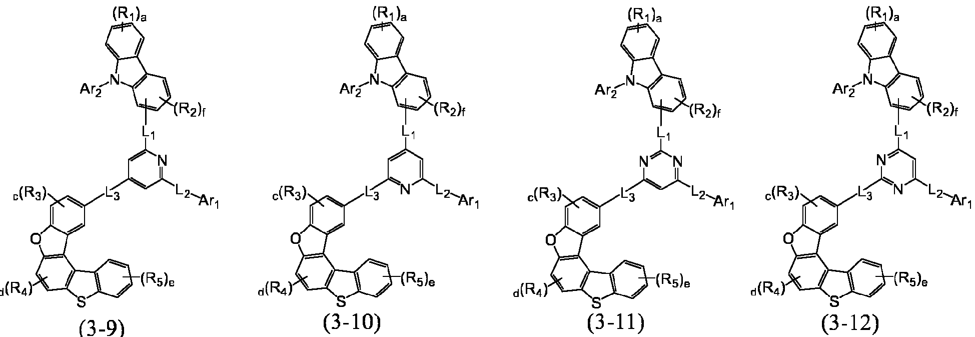

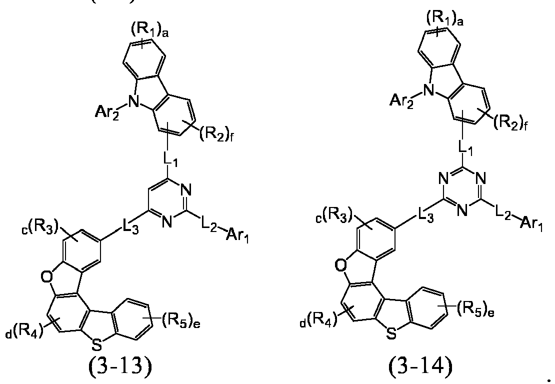

10. The mixture according to claim 8, wherein the L₁, L₂ and L₃ are each independently selected from a single bond, a substituted or unsubstituted phenyl, a substituted or unsubstituted biphenyl, a substituted or unsubstituted terphenyl, a substituted or unsubstituted pyridyl, a substituted or unsubstituted pyrimidinyl, a substituted or unsubstituted triazinyl, and a substituted or unsubstituted naphthyl; and a substituted substituent is selected from -D, or a linear alkyl group having 1 to 10 carbon atoms, or a branched or cyclic alkyl group having 3 to 10 carbon atoms, or a phenyl group, or a naphthyl group.

11. The mixture according to claim 8, wherein the heterocyclic group-containing organic compound structure is selected from one of formulas (4-1) to (4-8):

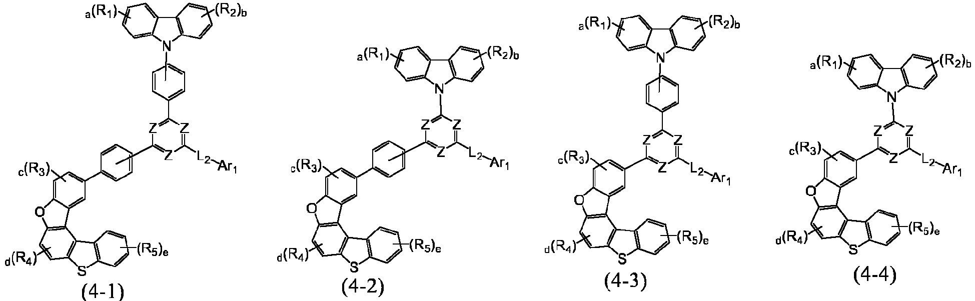

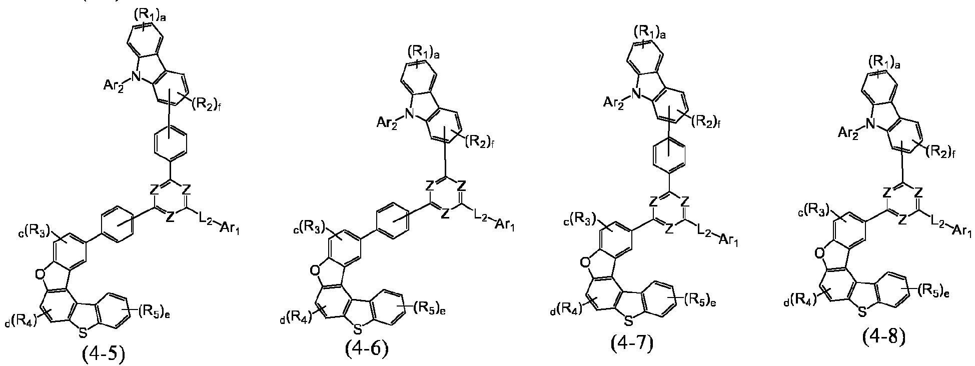

12. The mixture according to claim 8, wherein Ar₁ is selected from one of following structures or a combination thereof: 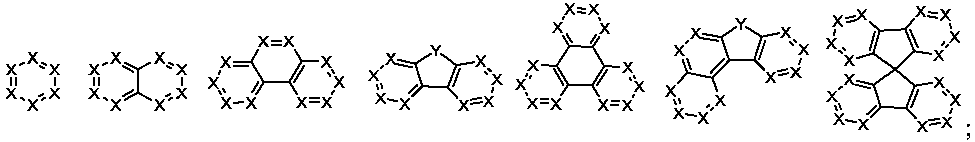 wherein:
X is independently selected from CR₇ or N;
Y is independently selected from C(R₈R₉), NR₈, Si(R₈R₉), O, S, Se, S=O, S(=O)₂, or PR₈;
R₇ to R₉ is independently selected from -H, -D, or a linear alkyl group having 1 to 20 carbon atoms, or a linear alkoxy group having 1 to 20 carbon atoms, or a linear thioalkoxy group having 1 to 20 carbon atoms, or a branched alkyl group having 3 to 20 carbon atoms, or a cyclic alkyl group having 3 to 20 carbon atoms, or a branched alkoxy group having 3 to 20 carbon atoms, or a cyclic alkoxy group having 3 to 20 carbon atoms, or a branched thioalkoxy group having 3 to 20 carbon atoms, or a cyclic thioalkoxy group having 3 to 20 carbon atoms, or a silyl group, or a ketone group having 1 to 20 carbon atoms, or an alkoxycarbonyl group having 2 to 20 carbon atoms, or an aryloxycarbonyl group having 7 to 20 carbon atoms, a cyano group, a carbamoyl group, a haloformyl group, a formyl group, an isocyano group, an isocyanate group, a thiocyanate group or an isothiocyanate group, a hydroxyl group, a nitro group, -CF₃, -Cl, -Br, -F, -I, or an aromatic group having 6 to 60 ring atoms, or a substituted aromatic group having 6 to 60 ring atoms, or a heteroaromatic group having 5 to 60 ring atoms, or a substituted heteroaromatic group having 5 to 60 ring atoms, or an aryloxy group having 6 to 60 ring atoms, or a heteroaryloxy group having 5 to 60 ring atoms, or a combinations thereof; and/or, adjacent R₇ form a ring with each other.

13. The mixture according to claim 8, wherein Ar₂ is selected from methyl, ethyl, n-propyl, isopropyl, n-butyl, sec-butyl, tert-butyl, isobutyl, phenyl, or naphthyl, or a combination thereof.

14. The mixture according to claim 8, wherein the heterocyclic group-containing organic compound is selected from any one of the following compounds:

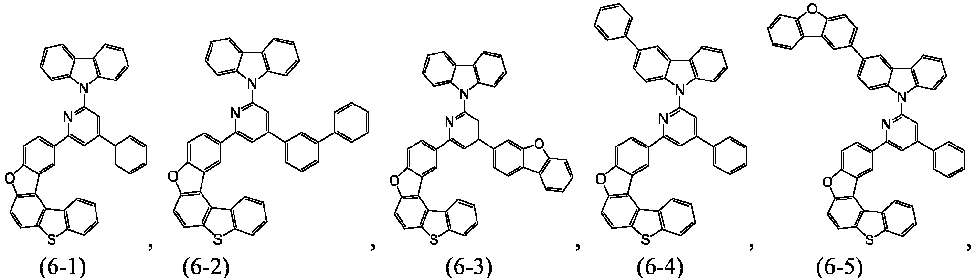

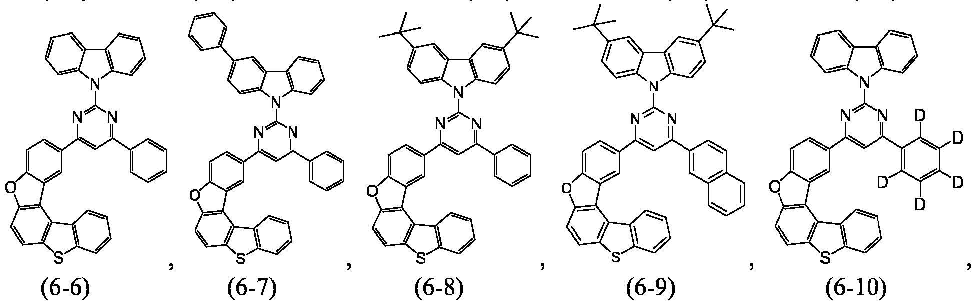

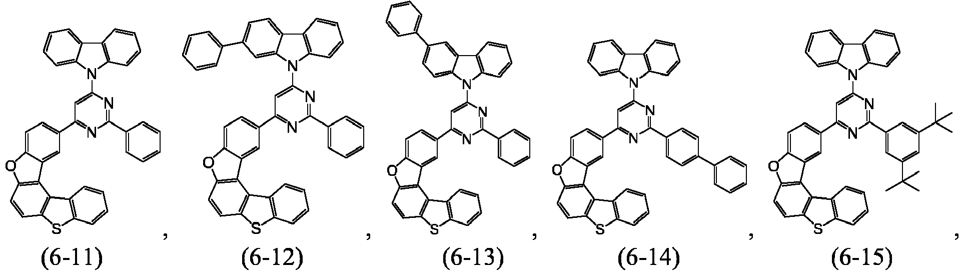

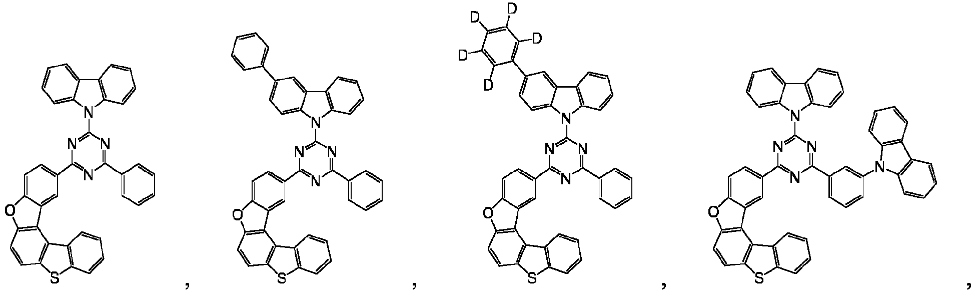

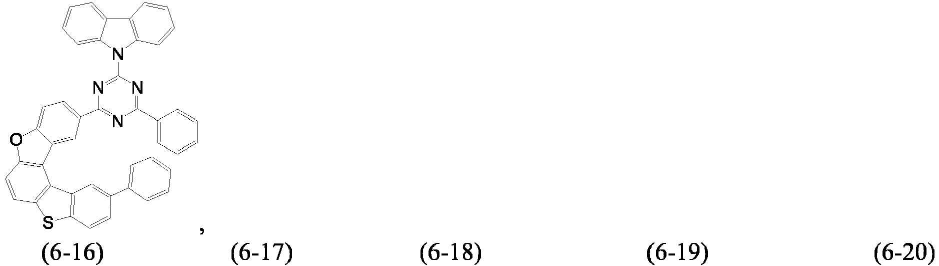

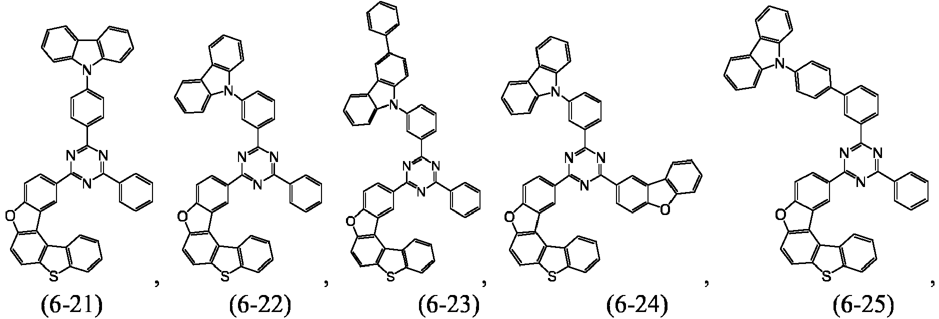

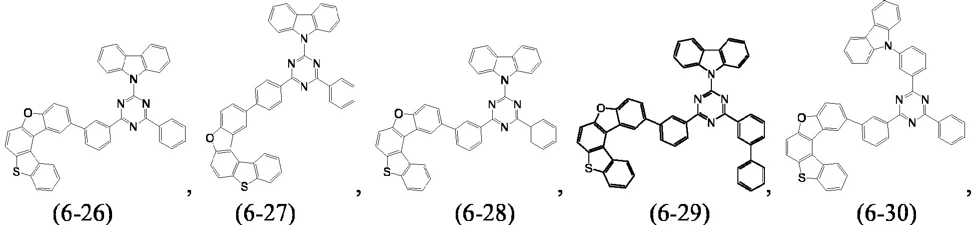

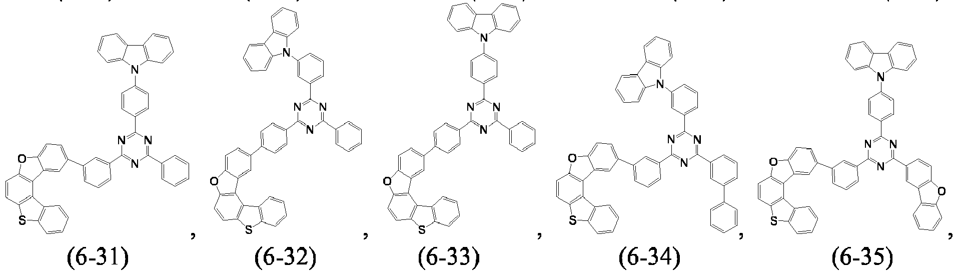

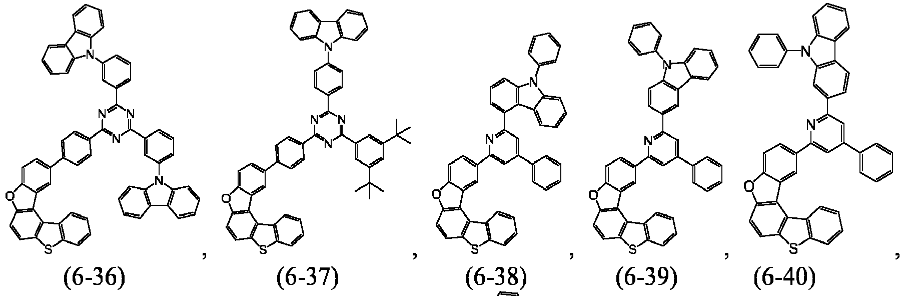

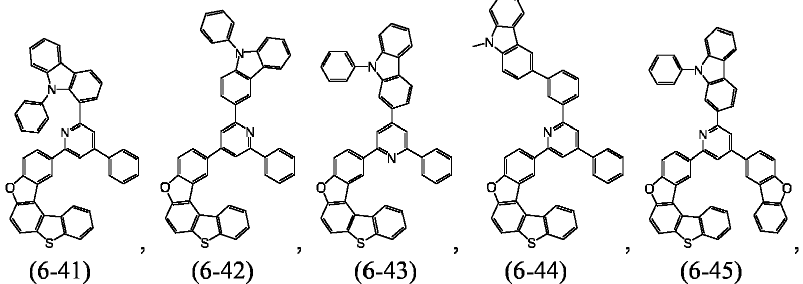

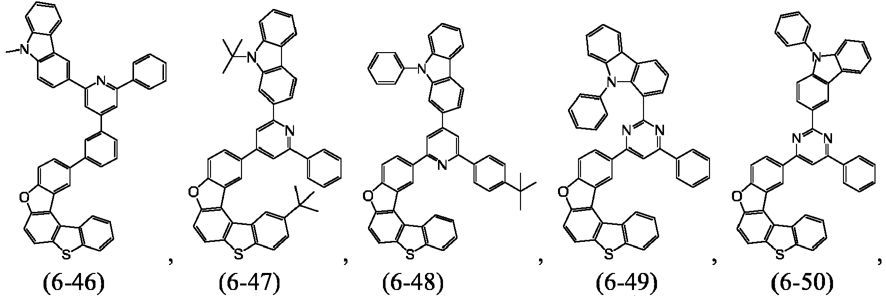

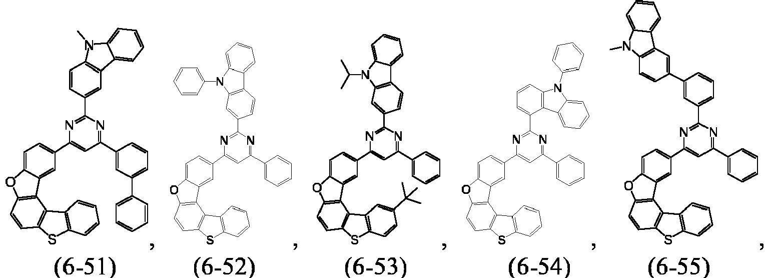

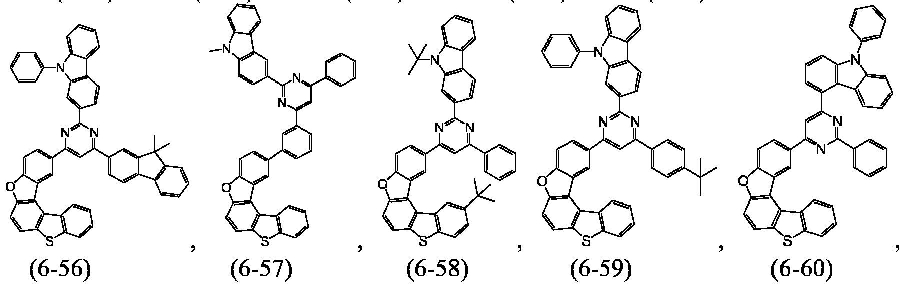

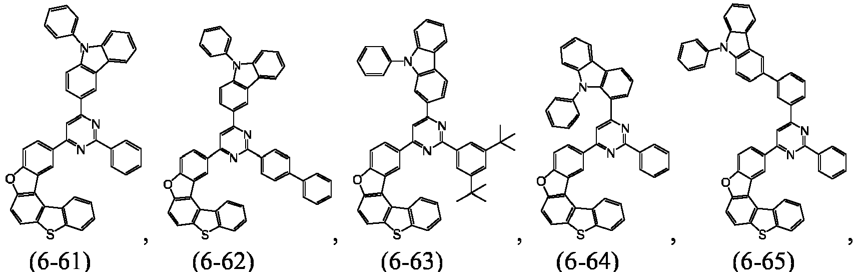

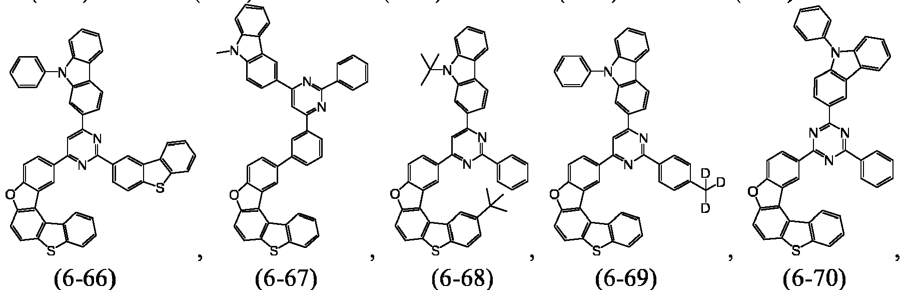

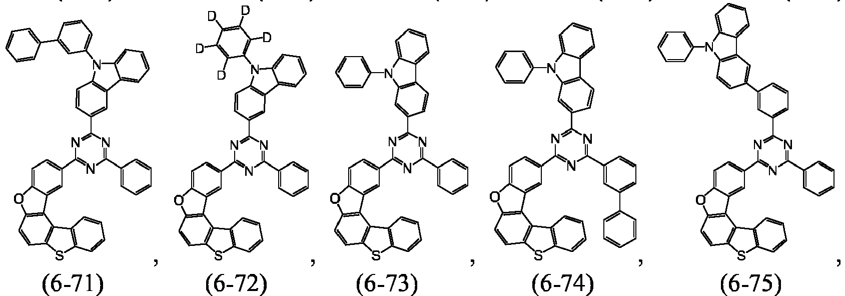

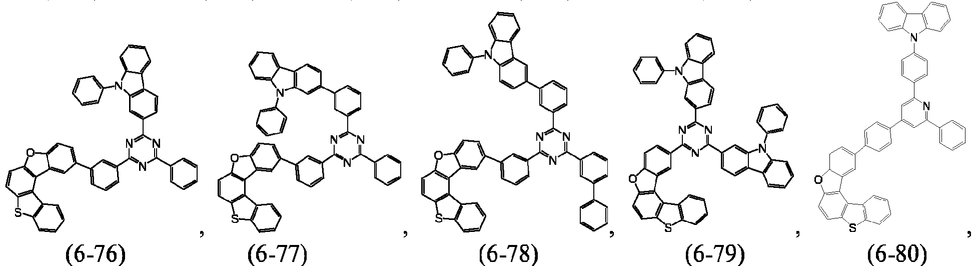

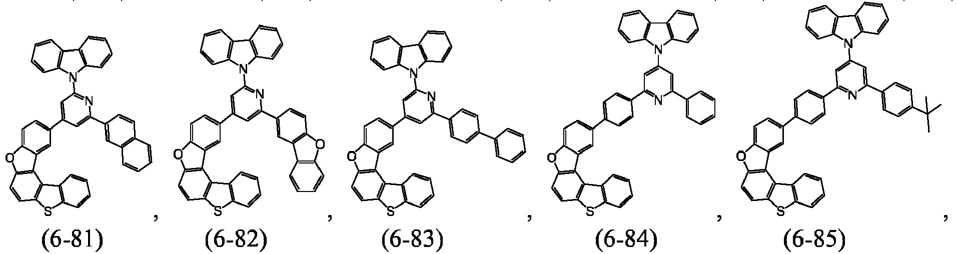

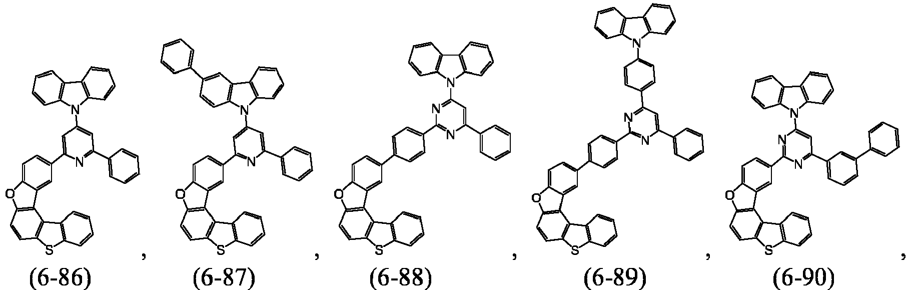

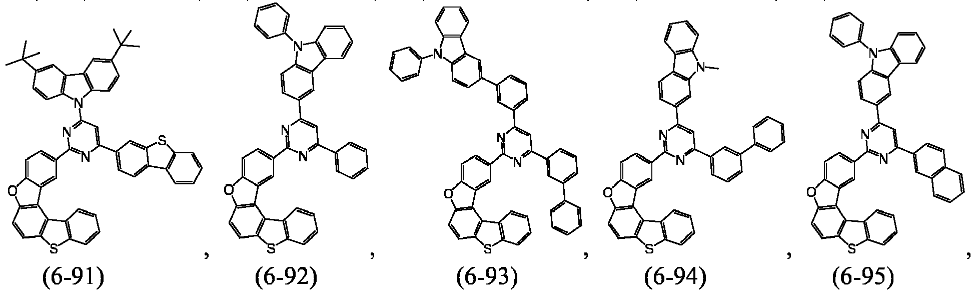

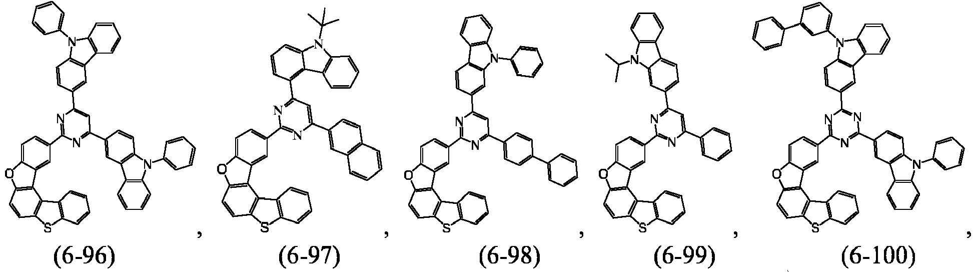

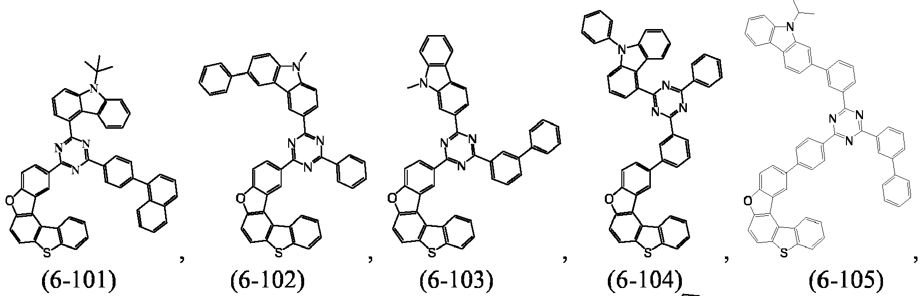

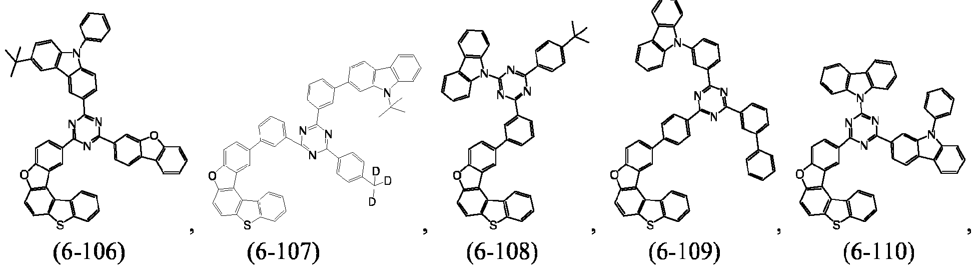

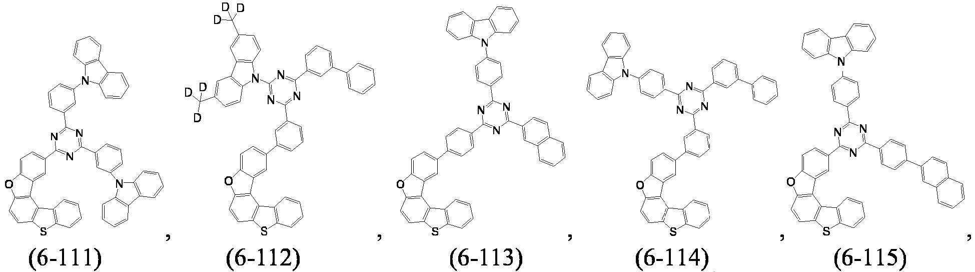

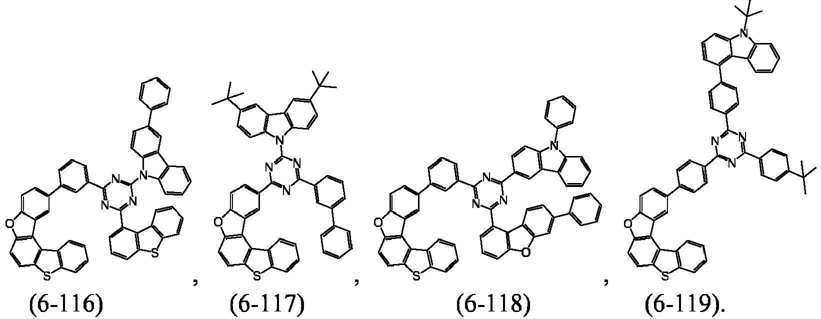

15. The mixture according to claim 8, wherein a molar ratio of the heterocyclic group-containing organic compound to the organic functional material ranges from 4:6 to 6:4.

16. The mixture according to claim 8, wherein the organic functional material has a structure selected from one of formulas (5-a) to (5-d): 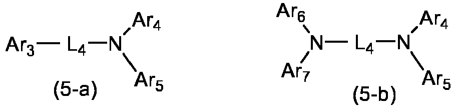 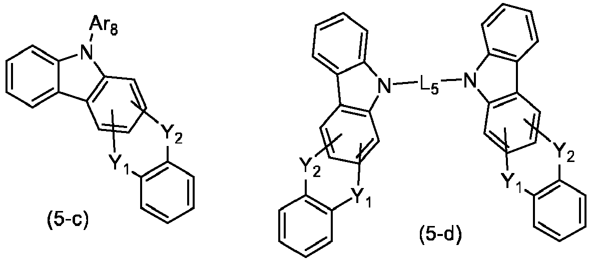
wherein Ar₃ to Ar₈ are each independently selected from a substituted or unsubstituted aromatic group having 6 to 30 ring atoms, or a substituted or unsubstituted heteroaromatic group having 5 to 30 ring atoms;
L₄ and L₅ are each independently selected from a substituted or unsubstituted aromatic group having 6 to 30 ring atoms, or a substituted or unsubstituted heteroaromatic group having 5 to 30 ring atoms;
Y₁ and Y₂ are each independently selected from C(R₁₀R₁₁), NR₁₀, O, S or a single bond; and
R₁₀ to R₁₁ are each independently selected from -H, -D, or a linear alkyl group having 1 to 20 carbon atoms, or a branched or cyclic alkyl group having 3 to 20 carbon atoms, or a substituted or unsubstituted aromatic or heteroaromatic group having 5 to 60 ring atoms.

17. The mixture according to claim 16, wherein
any two of Ar₄, Ar₅, L₄ form a ring with each other;
and/or any two of Ar₆, Ar₇, L₄ form a ring with each other;
and/or at least one H atom in the formula is substituted by a D atom.

18. The mixture according to claim 16, wherein the structure of the organic functional material is selected from one of formulas (5-f) to (5-i): 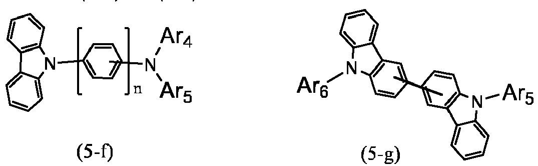 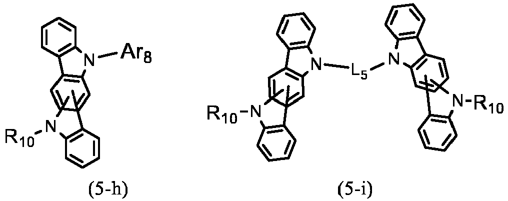 wherein n is selected from 0, 1, 2, 3 or 4.

19. An organic electronic device, wherein the organic electronic device comprises a first electrode, a second electrode, and at least one organic functional layer disposed between the first electrode and the second electrode, wherein the organic functional layer comprises at least one heterocyclic group-containing organic compound, and the heterocyclic group-containing organic compound has a structure represented by formula (1) or (2): 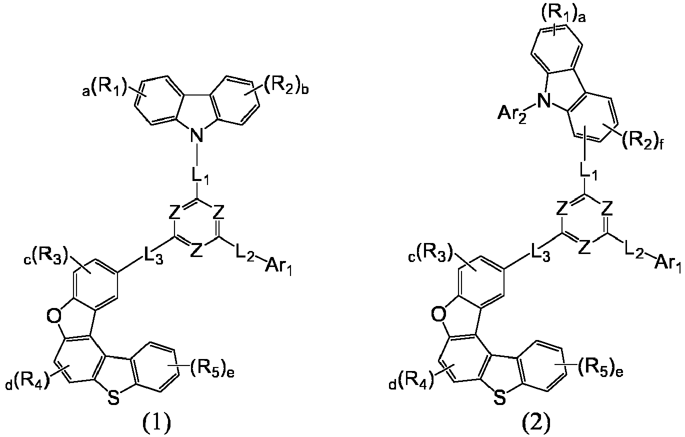 wherein:
Z is independently selected from CR₆ or an N atom, wherein at least one Z is an N atom;
R₁, R₂ or R₆ are each independently selected from -H, -D, or a linear alkyl group having 1 to 20 carbon atoms, a linear alkoxy group having 1 to 20 carbon atoms, or a linear thioalkoxy group having 1 to 20 carbon atoms, or a branched alkyl group having 3 to 20 carbon atoms, or a cyclic alkyl group having 3 to 20 carbon atoms, or a branched alkoxy group having 3 to 20 carbon atoms, or a cyclic alkoxy group having 3 to 20 carbon atoms, or a branched thioalkoxy group having 3 to 20 carbon atoms, or a cyclic thioalkoxy group having 3 to 20 carbon atoms, or a silyl group, or a ketone group having 1 to 20 carbon atoms, or an alkoxycarbonyl group having 2 to 20 carbon atoms, or an aryloxycarbonyl group having 7 to 20 carbon atoms, a cyano group, a carbamoyl group, a haloformyl group, a formyl group, an isocyano group, an isocyanate group, a thiocyanate group or an isothiocyanate group, a hydroxyl group, a nitro group, -CF₃, -Cl, -Br, -F, -I, or substituted or unsubstituted aromatic group having 6 to 60 ring atoms, or a substituted or unsubstituted heteroaromatic groups having 5 to 60 ring atoms, or a substituted or unsubstituted aryloxy group or heteroaryloxy group having 5 to 60 ring atoms, or a combinations thereof;
R₃, R₄ or R₅ are each independently selected from -H, -D, or a linear alkyl group having 1 to 20 carbon atoms, or a branched or cyclic alkyl group having 3 to 20 carbon atoms, or a phenyl group;
Ar₁ is selected from a substituted or unsubstituted aromatic group having 6 to 30 ring atoms, or a substituted or unsubstituted heteroaromatic group having 5 to 30 ring atoms;
Ar₂ is selected from -H, -D, or a linear alkyl group having 1 to 20 carbon atoms, or a branched or cyclic alkyl group having 3 to 20 carbon atoms, or a substituted or unsubstituted aromatic group having 6 to 30 ring atoms, or a substituted or unsubstituted heteroaromatic group having 5 to 30 ring atoms;
L₁, L₂ and L₃ are each independently selected from a single bond, or a substituted or unsubstituted aromatic group having 6 to 30 ring atoms, or a substituted or unsubstituted heteroaromatic group having 5 to 30 ring atoms; and
a, b and e are each independently selected from 0, 1, 2, 3, or 4; d is selected from 0, 1 or 2; and c and f are each independently selected from 0, 1, 2 or 3.

20. The organic electronic device according to claim 19, wherein the organic functional layer comprises a light-emitting layer, and the light-emitting layer comprises at least one of the heterocyclic group-containing organic compounds.
